# EUROPEAN PATENT APPLICATION

(11) **EP 3 915 363 A1**
(43) Date of publication of application: **01.12.2021**
(21) Application number: 20382459.4
(22) Date of filing: 29.05.2020
(51) Int. Cl.: A01K 67/027, C12N 5/10

(54) **R-RAS2 KNOCKOUT MOUSE MODEL OF MYELIN PATHOLOGIES**

(71) Applicant: Universidad Autónoma de Madrid, 28049 Madrid (ES)
(72) Inventor: CUBELOS ÁLVAREZ, Beatriz, 28049 Madrid (ES); ALCOVER SÁNCHEZ, Berta, 28049 Madrid (ES)
(74) Representative: Hoffmann Eitle

(57) **Abstract**

The present invention refers to a transgenic mouse model of myelin pathologies (or transgenic oligodendrocytes and/or neurons derived thereof). Moreover, the present invention also refers to the use of the mouse model of the invention (or oligodendrocytes and/or neurons derived thereof) in a method for screening candidate compounds for treating myelin pathologies.

## Description

### FIELD OF THE INVENTION

The present invention refers to the medical field. Particularly, it refers to a transgenic mouse model of myelin pathologies (or transgenic oligodendrocytes and/or neurons derived thereof). Moreover, the present invention also refers to the use of the mouse model of the invention (or oligodendrocytes and/or neurons derived thereof) in a method for screening candidate compounds for treating myelin pathologies.

### STATE OF THE ART

Myelin is critically required in the vertebrate nervous system to enable fast and efficient synaptic transmission. In the central nervous system (CNS), oligodendrocytes (OLs) create myelin and extend processes that wrap around axons and form compact myelin sheaths. These multilayered membrane compartments, or nodes, line up along the axon to provide metabolic support and protection from the extracellular space. Between nodal compartments lies Nodes of Ranvier, structures in which the molecular machinery responsible for electric transmission is concentrated. This organization enables fast saltatory conduction of action potentials initiated from the neuronal soma because axonal membrane depolarization skips from node to node, a feature believed to contribute to vertebrate evolution.

Myelin's role is so important that when it is impaired or lost, pathologies begin to appear. The onset of myelin dysfunction can impact a wide range of processes from embryonic development to chronic degenerative diseases such as multiple sclerosis. At a cellular level, loss of myelin causes channels responsible for depolarization to become distributed along the axon instead of being concentrated in Nodes of Ranvier, requiring greater number of depolarizations and increased use of ATP to fuel synaptic transmission. When whole-cell ATP levels are depleted, Na⁺/K⁺ ATPase is then unable to restore the Na⁺/K⁺ gradient after an action potential, which leads to degeneration and axonal dysfunction. To compensate for increased axonal energy demands, both numbers and activity of mitochondria in demyelinated areas increase, a well characterized feature of demyelinating diseases demonstrated both in humans and in numerous animal models.

Among the pathologies associated with myelin, sensorial, motor and cognitive alterations stand out. Some of the most important pathologies that affect the CNS are: multiple sclerosis (MS), neuromyelitis optica (NMO), hypomyelinating leukodystrophies and Charcot-Marie-Tooth disease (CMT). For example, in the case of Multiple Sclerosis alone, 1,800 new cases are diagnosed each year in Spain alone. Multiple sclerosis affects 47,000 people in Spain, 700,000 people in Europe and 2.5 million people worldwide. In the past two decades, the number of patients with multiple sclerosis has doubled. The average delay in the diagnosis and treatment of multiple sclerosis is between one and two years. Multiple sclerosis is the second cause of disability among Spanish youth, after traffic accidents.

Multiple sclerosis is the most prevalent CNS neurological disease that affects young adults. In fact, it is generally diagnosed at the age of 20-40 years and is approximately 2 or 3 times more frequent in women than in men. The etiology remains unknown; however, it is believed to occur as a result of some combination of genetic and environmental factors. Today, MS is considered a chronic, autoimmune, and neurodegenerative disease that affects more than 2 million people worldwide. This neurodegenerative disease is characterized by demyelination with concomitant axonal and neuronal degeneration that causes a heterogeneous variety of symptoms and signs.

Another neurodegenerative disease is neuromyelitis optica (NMO, previously called Devic's disease). It is considered a rare, autoimmune, demyelinating CNS disease that has optic neuritis and manifestations of acute transverse myelitis. This disease affects more than 2 million people worldwide and has a prevalence of 1-3 per 100,000 and is more common in women than in men (from 2: 1 to 10: 1). It was first considered as a monophasic syndrome that was a subtype of MS until the discovery of autoantibodies against aquaporin-4 (AQP4-IgG). Furthermore, among patients with NMO there is a seronegativity of 10% to 25% for AQP4-IgG, which suggests the participation of other factors in the development of the disease, such as autoantibodies against myelin oligodendrocytic glycoprotein (MOG-IgG).

On the other hand, leukodystrophies are rare diseases, often genetic, characterized by a loss of myelin in the CNS and PNS. They have very diverse origins, such as mutations that affect different cellular compartments, cellular processes, or protein translation. Only some genes whose mutations cause leukodystrophies encode myelin diseases, such as Pelizaeus-Merzbacher disease (PMD) where the PLP1 gene is mutated.

Another neurodegenerative disease is Charcot Marie-Tooth disease classified as sensory and motor neuropathy. It is a phenotypic and genetically heterogeneous group of classically primary genetic neuropathies. CMT is the most common hereditary neuropathy worldwide with a prevalence of 1: 2,500, and it is a childhood-onset syndrome. It has enormous heterogeneity due to the different inheritance patterns (autosomal dominant, autosomal recessive, X-linked) classified into different electrophysiological classes characterized by electromyography (axonal, demyelinating, intermediate). The most frequent pattern is autosomal dominant inheritance.

Despite the advances in the study of myelin diseases (for instance amyotrophic lateral sclerosis, neuromyelitis optica, multiple sclerosis, Charcot Marie Tooth disease or leukodystrophies), it is still needed a greater insight from a neurological perspective. Nevertheless, in order to gain insight into these human diseases it is important to generate animal models which faithfully reproduce the human condition. This would give the researchers the possibility of better understanding these pathological conditions and assaying effective treatments.

Currently there are two animal models for the study of myelin diseases. Both models are based on the artificial aggression of the animal's immune system in order to reproduce the immune symptoms:
- Immune (EAE): An autoimmune reaction is caused by direct immunization after injection of myelin proteins. This generates an immune response of the animal against these injected foreign antigens. This system is the Experimental Autoimmune Encephalomyelitis (EAE) model. In this model, the animal's immune system produces antibodies that attack and destroy myelin.
- Viral: This model is an inoculum of coronavirus (murine hepatitis virus), enterovirus (Theiler virus), lentivirus or canine distemper virus. The most widespread is the model based on inoculation of Theiler virus that produces murine demyelinating encephalomyelitis caused by Theiler virus (TMEV-IDD Theiler murine encephalomyelitis virus-induced demyelinated disease). These models are included in the autoimmune reaction caused by the injection of viruses that in an unknown way presented an immune response of the animal against the myelin antigens due to the similarity it possesses with the virus's own antigens.

Currently, these models are used to validate the therapeutic effect of new compounds. However, although some compounds have been effective in these models, this effect was not been reproduced in preclinical trials (including aggravated symptoms such as IFN-gamma). Therefore, the development and improvement of animal models which faithfully reproduce the pathophysiology of these diseases is of utmost importance. New models that reproduce the symptomatology of these diseases from a neurological point of view is therefore a priority of crucial importance because this would allow the study of the intrinsic factors that can elucidate possible treatments for these diseases.

The present invention is thus focused on solving this problem and it is herein proposed a transgenic mouse model of myelin pathologies which faithfully resembles the human condition, and a method for screening candidate compounds for treating myelin pathologies.

### DESCRIPTION OF THE INVENTION

The inventors of the present invention have developed a new model that faithfully reproduces the symptomatic characteristics of myelin pathologies from a neurological point of view. This is highly relevant because myelin pathologies are in fact neurodegenerative diseases. What is even more relevant is that the animal models of the present invention do not have a functional immune system, so this component would not be decisive in finding a treatment. This means that the capacity of drugs to myelinate could be really evaluated in physiological conditions very similar to those presented in these diseases.

The inventors of the present invention have discovered that *R-Ras1* and/or *R-Ras2* loss of function is associated with aberrantly myelinated axons with increased numbers of mitochondria and a disrupted mitochondrial respiration that leads to increased ROS levels. As a consequence, aberrantly myelinated axons are thinner with cytoskeletal phosphorylation patterns typical of axonal degeneration processes characteristic of myelin diseases. Although different levels of hypomyelination in single mutant mice were observed, the combined loss of function in DKO mice lead to a compromised axonal integrity triggering a loss in visual function. Thus, the present invention shows that *R-Ras* loss of function reproduces several characteristics of myelin diseases, and it is herein proposed that *R-Ras1*^{*-*/*-*} and/or *R-Ras2*^{*-*/*-*} neurological models are valuable approaches for the study of myelin pathologies.

Particularly, the present invention shows that loss of function of small GTPases and oncogenes *R-Ras1* and *R-Ras2* in mice result in a hypomyelination phenotype reminiscent of myelinating disorders such as multiple sclerosis. Specifically, the inventors of the present invention found evidence of disrupted mitochondrial function and elevated ROS levels and that mutant mice suffered from axonal degeneration that led to visual loss. These findings point to the possibility of using R-Ras mutant mice to model myelinating disorders and assess potential therapeutic agents for their capacity to reverse these phenotypes.

Consequently, the first embodiment of the present invention refers to a transgenic knock-out *R-Ras2*^{-/-} mouse model of myelin pathologies, or transgenic oligodendrocytes and/or neurons derived thereof, characterized by the disruption or inactivation of the gene *R-Ras2.*

Preferably, the present invention refers to a transgenic double knock-out *R-Ras1*^{-/-} and *R-Ras2*^{-/-} mouse model of myelin pathologies, or transgenic oligodendrocytes and/or neurons derived thereof characterized by the disruption or inactivation of the genes *R-Ras2* and *R-Ras1.*

Kindly note that *R-Ras2*^{-/-} mouse model does not have a competent immune system, so it allows to study the neurological role in the absence of immune disturbance. On the other hand, *R-Ras2*^{*-*/*-*} (and *R-Ras1*^{-/-} and *R-Ras2*^{-/-}*)* mouse provides a model that affects oligodendrocytes-neuron interaction never described before.

In the absence of *R-Ras2,* or in the absence of *R-Ras1* and *R-Ras2,* the following characteristics of demyelinating diseases are obtained:
- Hypomyelination of the main myelinated tracts of the CNS caused by the immaturity of the oligodendrocyte. This hypomyelination is moderate in the absence of *R-Ras2* and severe in the absence of *R-Ras1* and *R-Ras2.*
- The absence of *R-Ras2,* or *R-Ras1* and *R-Ras2,* produces immaturity of oligodendrocytes and neurons.
- Mitochondrial alterations are also produced in neurons:
   ∘ Moderate modifications: Increased number of mitochondria in neurons lacking R-*Ras2.*
   ∘ Very severe modifications: Increased number of mitochondria, increased number of mitochondrial complexes, and increased mitochondrial activity in neurons lacking *R-Ras1* and *R-Ras2.*
- Since the absence of *R-Ras1* and/or *R-Ras2* does not produce mitochondrial alterations in neurons or oligodendrocytes separately, the essential role of the oligodendrocyte-neuron interaction is herein demonstrated.
- Metabolic disturbances typical of demyelinating diseases:
   ∘ Pyruvate / lactate ratio alterations indicating moderate mitochondrial alteration in R-Ras2^{-/-} and severe mitochondrial alteration in R-Ras1^{-/-} and R-Ras2^{-/-}.
   ∘ Alterations in the amount of metabolites directly related to mitochondrial activity.
- Alterations of neuron integrity:
   ∘ Moderate increase in ROS in the case of R-Ras2^{-/-} and more severe in model R-Ras1^{-/-} and R-Ras2^{-/-}.
   ∘ Alteration of the integrity of the axonal cytoskeleton, which maintains the stability of the neuron's function.
- Typical alterations of neuron degeneration in demyelinating diseases:
   ∘ Increase of typical markers of neuron degeneration.
   ∘ Expression of molecules that prevent myelination.

Thus, the proposed models reproduce the symptoms of demyelinating diseases from a neurological approach. It is important to note that *R-Ras2*^{-/-} model reproduces pathologies to a moderate degree, while double mutant *R-Ras1*^{-/-} and *R-Ras2*^{-/-} reproduces diseases to a severe degree.

Particularly, *R-Ras2*^{-/-} model has some important characteristics to be a good model, such as: Decrease of oligodendrocyte population (30%), decreased activation of signalling pathways, moderate decrease in myelin levels (30%), loss of the myelin sheath surrounding the axons (30%), oligodendrocyte immaturity, neuron degeneration , slowdown of the transmission rate of the nerve impulse, mitochondrial increase in neurons, alterations in the Pyruvate/Lactate ratio (indicator of mitochondrial / demyelinating pathology), cognitive deficit and motor deficit. In the context of the present invention, the following characteristics are particularly relevant: neuron degeneration, mitochondrial increase in neurons, alterations in the Pyruvate/Lactate ratio (indicator of mitochondrial / demyelinating pathology), cognitive deficit and motor deficit.

On the other hand, double mutant *R-Ras1*^{-/-} and *R-Ras2*^{-/-} model has improved characteristics over the single mutant, such as: Decrease in the oligodendrocyte population (55%), decreased activation of signalling pathways (50%), dramatic decrease in myelin levels (60%), loss of the myelin sheath surrounding the axons (80%), oligodendrocyte immaturity, neuron degeneration (neuron expression of the neuronal immaturity marker PSA-NCAM), Ranvier node alterations, slowdown of the transmission rate of the nerve impulse, mitochondrial increase in the neurons, expression increase of mitochondrial complexes in oligodendrocytes / neurons, mitochondrial activity increase, changes in the Pyruvate/Lactate ratio (indicator of mitochondrial / demyelinating pathology), increases in ROS levels, increased axonal calibre, alterations in the cytoskeleton of the neuron axon, cognitive deficit and motor deficit. In the context of the present invention, the following characteristics are particularly relevant: neuron degeneration (neuron expression of the neuronal immaturity marker PSA-NCAM), Ranvier node alterations, slowdown of the transmission rate of the nerve impulse, mitochondrial increase in neurons, expression increase of mitochondrial complexes in oligodendrocytes / neurons, mitochondrial activity increase, changes in the Pyruvate/Lactate ratio (indicator of mitochondrial / demyelinating pathology), increases in ROS levels, increased axonal calibre, alterations in the cytoskeleton of the neuron axon, cognitive deficit and motor deficit.

The second embodiment of the present invention refers to an *in vitro* method for screening candidate compounds for treating myelin pathologies, which comprises: a) determining in the mouse model or the oligodendrocytes and/or neurons of the invention the level of expression of *R-Ras2* after administering the candidate molecule and b) where, if after administering the candidate molecule, the level of expression of *R-Ras2* is higher as compared with the level of expression determined before administering the candidate molecule, this is indicative that the candidate molecule is effective in the treatment myelin pathologies.

In a preferred embodiment, the *in vitro* method for screening candidate compounds for treating myelin pathologies, comprises: a) determining in the mouse model or the oligodendrocytes of the invention the level of expression of *R-Ras2* and *R-Ras1* after administering the candidate molecule and b) where, if after administering the candidate molecule, the level of expression of *R-Ras2* and *R-Ras1* is significantly higher as compared with the level of expression determined before administering the candidate molecule, this is indicative that the candidate molecule is effective in the treatment myelin pathologies.

In a preferred embodiment, the *in vitro* method for screening candidate compounds for treating myelin pathologies, further comprises determining whether the oligodendrocytes and/or neurons have evolved to mature oligodendrocytes and/or neurons, wherein if immature oligodendrocytes and/or neurons have evolved to mature oligodendrocytes and/or neurons after administering the candidate molecule, this is indicative that the candidate molecule is effective in the treatment myelin pathologies. The determination of the maturity degree of the oligodendrocytes/neurons can be carried out by using the common general knowledge in this field, particularly by determining the expression level of the following biomarkers: PDGFRalfa, A2B5, NG2, PSA-NCAM, O4, NKX2 and/orTCF7L2, wherein an increased level of expression of these biomarkers is indicative that the oligodendrocytes and/or neurons have evolved to mature oligodendrocytes and/or neurons.

In a preferred embodiment, the *in vitro* method for screening candidate compounds for treating myelin pathologies, further comprises determining whether the oligodendrocytes and/or neurons are able to generate myelin, wherein if the oligodendrocytes and/or neurons are able to generate myelin, this is indicative that the candidate molecule is effective in the treatment myelin pathologies. Determining whether oligodendrocytes and/or neurons are able to generate myelin can be carried out by using the common general knowledge in this field, particularly by determining the expression level of the following biomarkers: MBP, MAG, MOG, PLP and/or CNPasa, wherein an increased level of expression of these biomarkers is indicative that the oligodendrocytes and/or neurons are able to generate myelin.
The third embodiment of the present invention refers to an *in vitro* method for obtaining a transgenic mouse model of myelin pathologies, or transgenic oligodendrocytes and/or neurons derived thereof, which comprises the disruption or inactivation of the gene *R-Ras2.* In a preferred embodiment the method father comprises the disruption or inactivation of the gene *R-Ras1.*

In a preferred embodiment of the invention, the myelin pathology selected from the group comprising: amyotrophic lateral sclerosis, neuromyelitis optica, multiple sclerosis, Charcot Marie Tooth disease and leukodystrophies.

For the purpose of the present invention the following terms are defined:
- The term "comprising" includes, but it is not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.

### Description of the figures

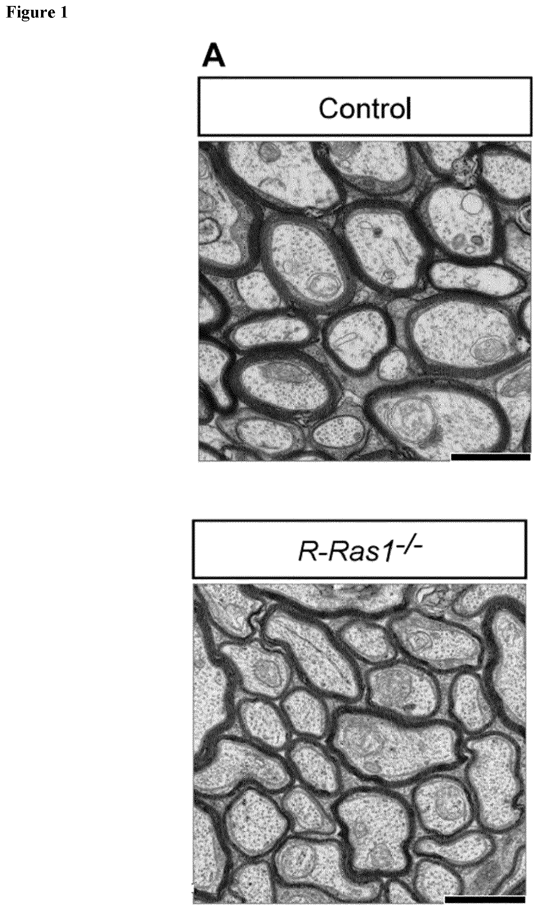

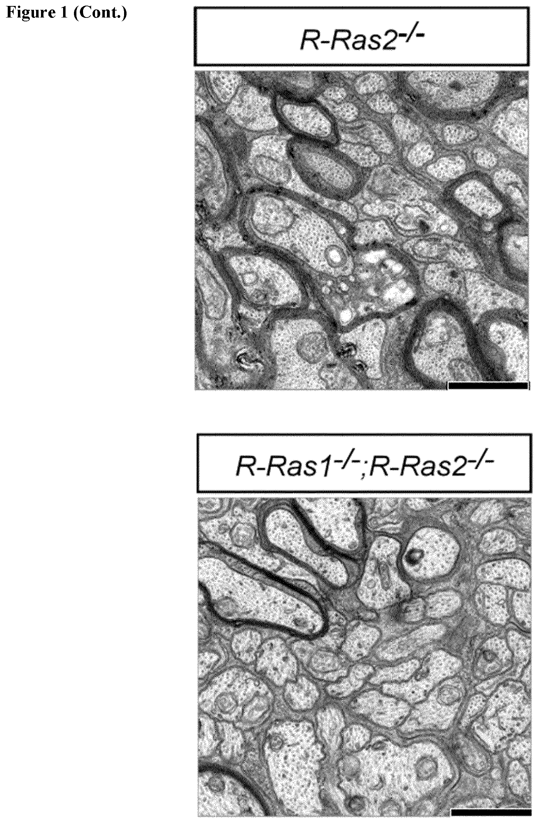

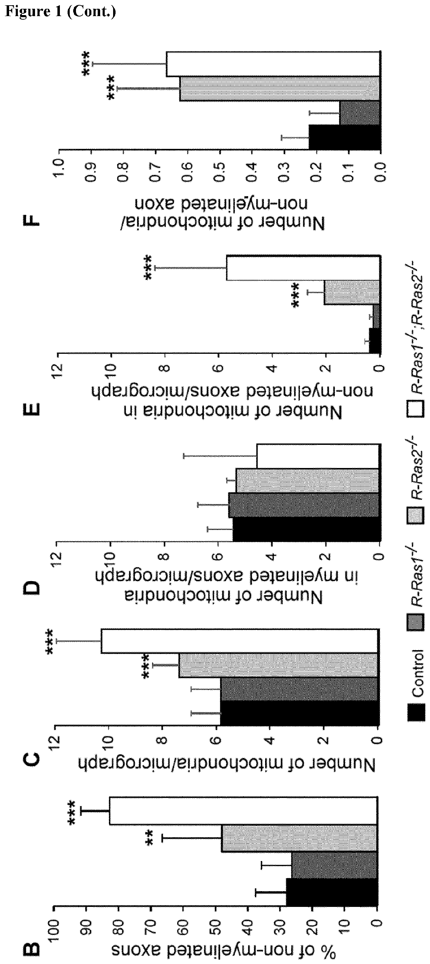

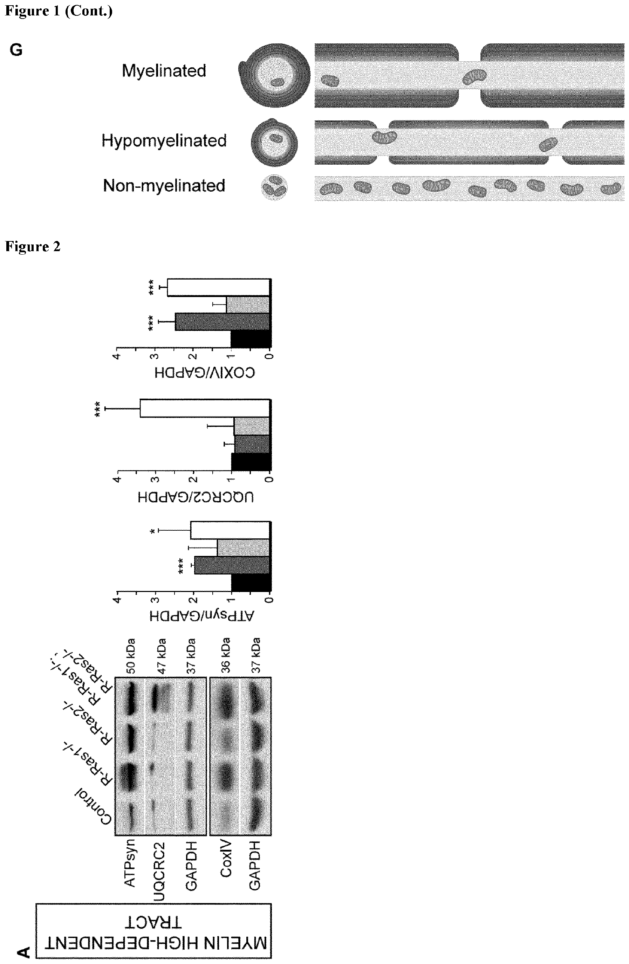

**Figure 1****. Loss of R-Ras2 affects axonal myelination of optic nerves, causing an increase in axonal mitochondria. *A***, Electron microscopy of transverse sections of the optic nerve from adult (P90) control, *R-Ras1*^{-/-} *R-Ras2*^{-/-} and *R-Ras1*^{-/-}*;R-Ras2*^{-/-} double knockout mice. There was a significant increase of non-myelinated axons in absence of R-Ras2 in single and double-mutants compared to controls. ***B***, Percentage of non-myelinated axons present in the optic nerve from adult mice (P90). Bar graph represents mean ± SD of control 27.87 ± 9.82%, *R-Ras1*^{-/-} 26.34 ± 9.13%, *R-Ras2*^{-/-} 47.98 ± 17.24%, and *R-Ras1*^{-/-}*;R-Ras2*^{-/-} 82.76 ± 8.81% showed significant differences in *R-Ras2*^{-/-} (***p* < 0.01) and the double-mutant mice (****p* < 0.001) compared to the control. ***C***, Number of mitochondria per micrograph present in each electron micrograph of optic nerve from adult mice (P90). Bar graph represents mean ± SD of *R-Ras1*^{-/-} 5.83 ± 1.11, *R-Ras2*^{-/-} 7.37 ± 0.98, and *R-Ras1*^{-/-}*;R-Ras2*^{-/-} 10.25 ± 1.68 compared to control 5.81 ± 1.12, showed significant differences observed in *R-Ras2*^{-/-} (****p* < 0.001) and the double-mutant mice (****p <* 0.001). ***D**,* Number of mitochondria in myelinated axons per micrograph present in optic nerve from adult mice (P90) did not reveal any differences between simple and double-mutants relative to controls. Bar graph represents mean ± SD of control 5.44 ± 0.96, *R-Ras1*^{-/-} 5.59 ± 1.17, *R-Ras2*^{-/-} 5.32 ± 0.36 and *R-Ras1*^{-/-}*;R-Ras2*^{-/-} 4.55 ± 2.72. ***E***, Number of mitochondria in non-myelinated axons per micrograph present in optic nerve from adult mice (P90). Bar graph represents mean ± SD of control 0.37 ± 0.18, *R-Ras1*^{-/-} 0.24 ± 0.14, *R-Ras2*^{-/-} 2.05 ± 0.64 and *R-Ras1*^{-*l*-}*;R-Ras2*^{-/-} 5.71 ± 2.67. This data showed significant differences in R-*Ras2*^{-/-} (****p <* 0.001) and the double-mutant mice (****p <* 0.001). ***F***, Average number of mitochondria per non-myelinated axon present in optic nerve from adult mice (P90). Bar graph represents mean ± SD of control 0.22 ± 0.09, *R-Ras1* ^{-/-} 0.13 ± 0.09, *R-Ras2*^{-/-} 0.62± 0.20 and *R-Ras1*^{-/-}*;R-Ras2*^{-/-} 0.67 ± 0.23. This data showed significant differences in *R-Ras2*^{-/-} (****p <* 0.001) and the double-mutant mice (****p* < 0.001). ***G***, Schematic representation of mitochondria number in longitudinal and transversal sections of axons with myelin alterations. All samples were obtained from adult mice (P90) (n = 3). Scale bar, 500 µm.

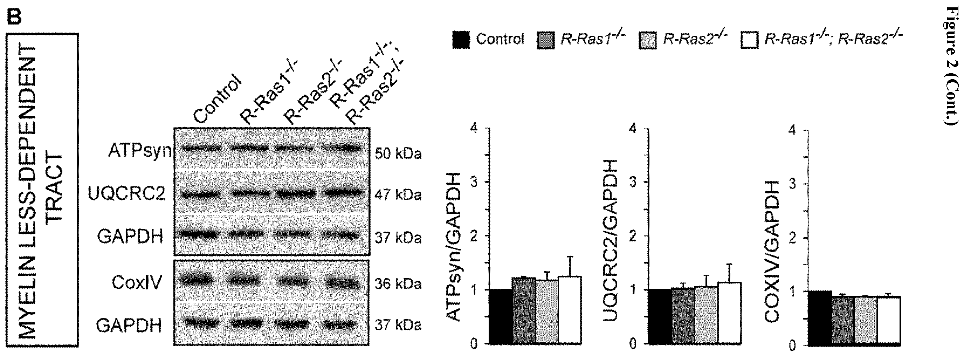

**Figure 2****. Expression of mitochondrial complexes III, IV and V are increased in the absence of R-Ras1. *A***, Western blot analysis of ATP synthase (Complex V), UQCRC2 (Complex III) and COXIV (Complex IV) in optic nerve lysates from adult mice (P90) showed an increment on the expression of these mitochondrial complexes in *R-Ras1*^{-/-} simple and double-mutants compared to control in myelin dependent tract. Quantification of western blot evidenced significant increase of mitochondrial Complexes III, IV and V in *R-Ras1*^{-/-} simple and double-mutant mice compared to control. Bar graphs represent mean ± SD of the fold-change relative to the WT of measurements normalized to GAPDH. ATP synthase: *R-Ras1*^{-/-} 1.97 ± 0.09, *R-Ras2*^{-/-} 1.37 ± 0.76 and *R-Ras1*^{-/-}*;R-Ras2*^{-/-} 2.08 ± 0.84. This data showed significant differences in *R-Ras1*^{-/-} (****p <* 0.001) and the double-mutant mice (****p <* 0.001) *(n =* 3). UQCRC2: *R-Ras1*^{-/-} 0.91 ± 0.29, *R-Ras2*^{-/-} 0.93 ± 0.70 and *R-Ras1*^{-/-}*;R-Ras2*^{-/-} 3.40 ± 0.92. This data showed significant differences in the double-mutant mice (****p <* 0.001) compared to the controls *(n* = 3). COXIV: *R-Ras1*^{-/-} 2.45 ± 0.45, *R-Ras2*^{-/-} 1.13 ± 0.36 and *R-Ras1*^{-/-}*;R-Ras2*^{-/-} 2.67 ± 0.21. This data showed significant differences in *R-Ras1*^{-/-} (****p <* 0.001) and the double-mutant mice (****p <* 0.001) *(n* = 3). ***B***, Western blot analysis of ATP synthase (Complex V), UQCRC2 (Complex III) and COXIV (Complex IV) in cerebral cortex lysates of adult mice (P90) showed no differences on the expression of these mitochondrial complexes in myelin less-dependent tract. Quantification of western blot didn't show any difference of mitochondrial Complexes III, IV and V in simple and double-mutant mice compared to control. Bar graph represents mean ± SD of the change relative to the control of measurements normalized to GAPDH. ATP synthase: R-*Ras1*^{-/-} 1.22 ± 0.02, *R-Ras2*^{-/-} 1.18 ± 0.15 and *R-Ras1*^{-/-}*;R-Ras2*^{-/-} 1.24 ± 0.36 *(n* = 3). UQCRC2: *R-Ras1*^{-/-} 0.91 ± 0.04, *R-Ras2*^{-/-} 0.91 ± 0.02 and *R-Ras1*^{-/-}*;R-Ras2*^{-/-} 0,87 ± 0.21 *(n* = 3). COXIV: *R-Ras1*^{-/-} 2.45 ± 0.45, *R-Ras2*^{-/-} 1.13 ± 0.36 and *R-Ras1* ^{-/-}*;R-Ras2* ^{-/-}2.67 ± 0.21 *(n* = 3).

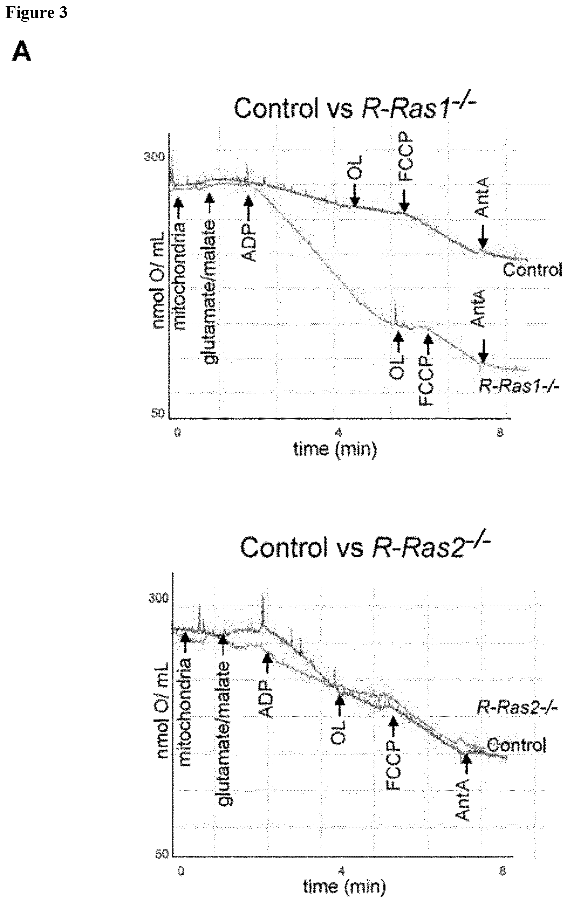

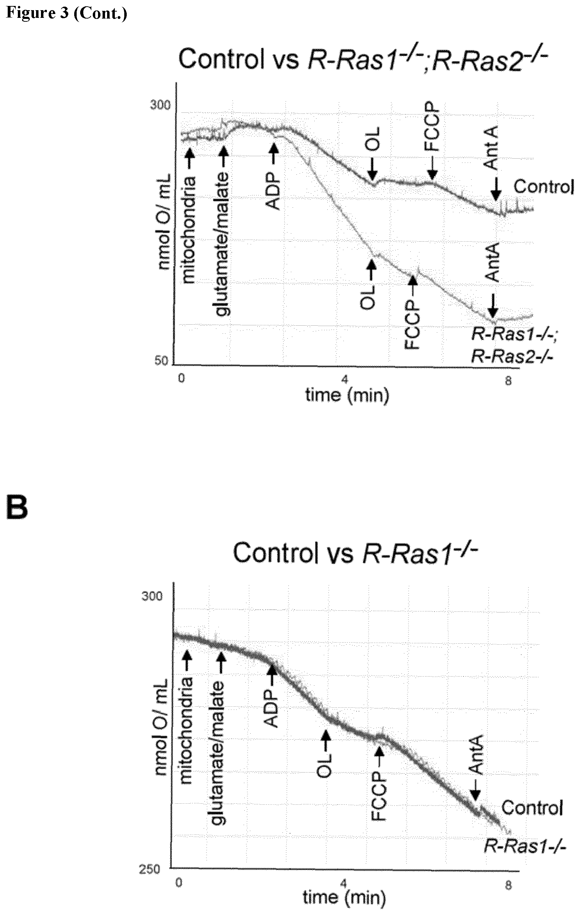

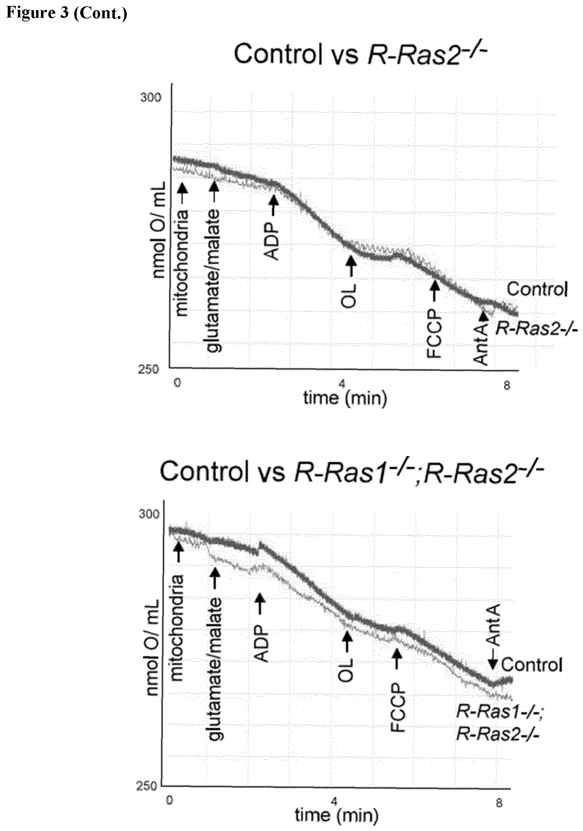

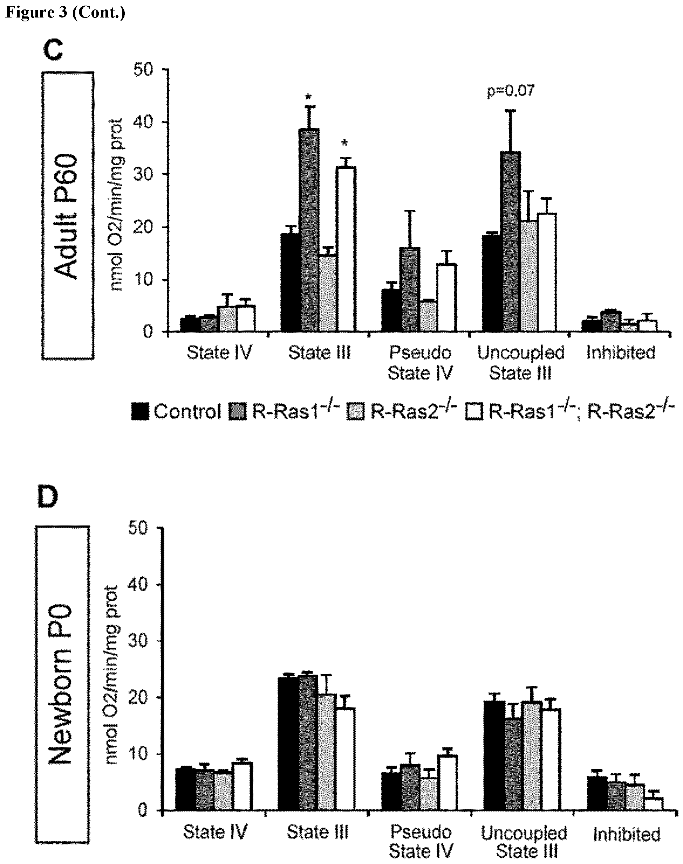

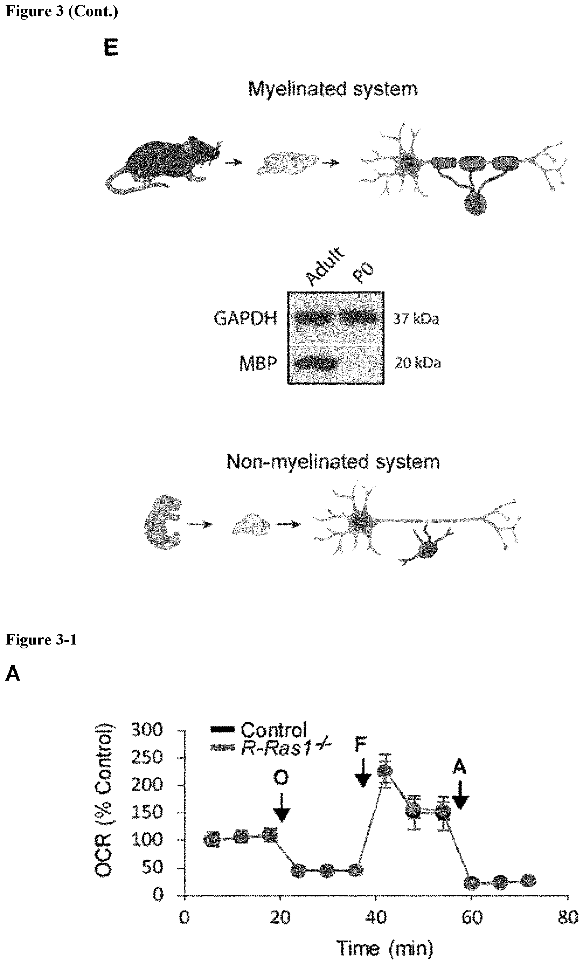

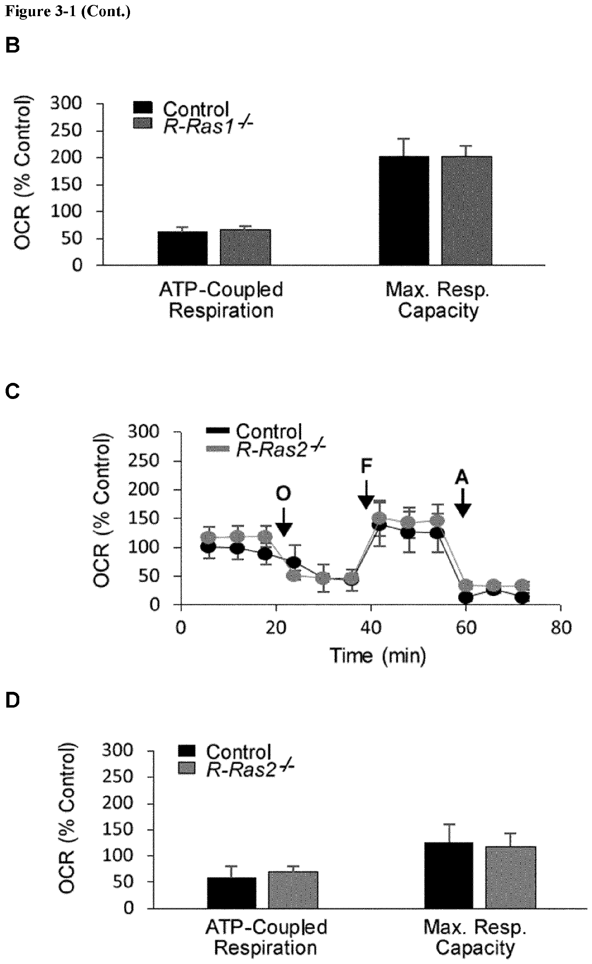

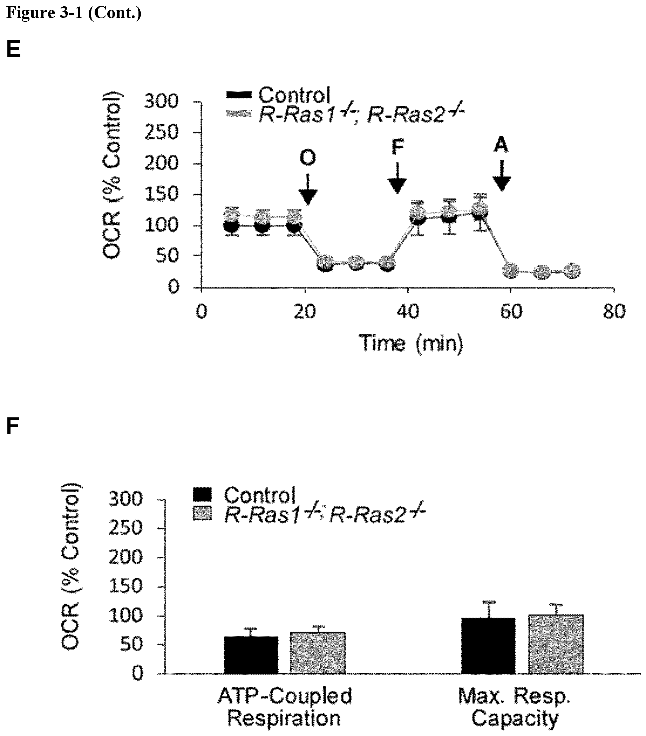

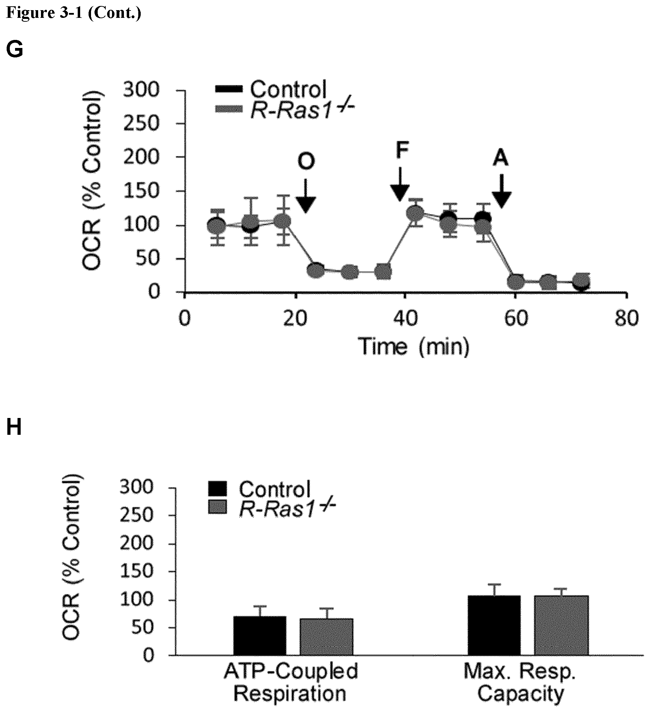

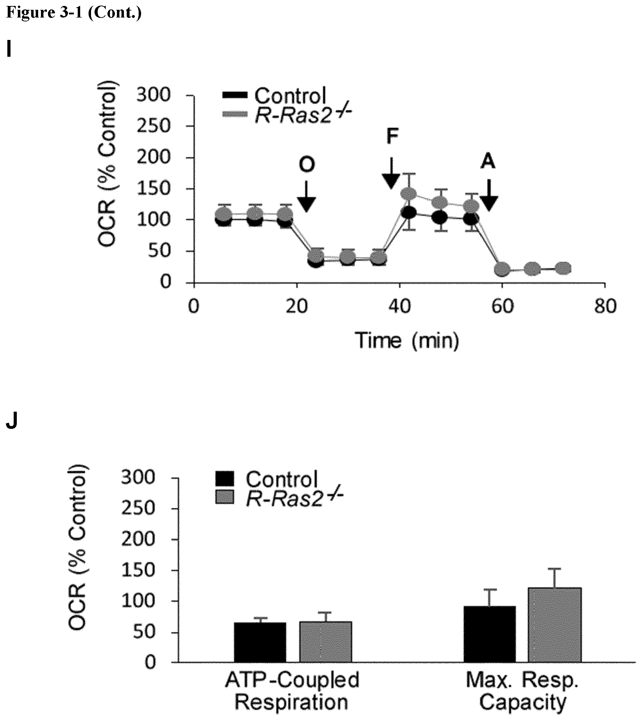

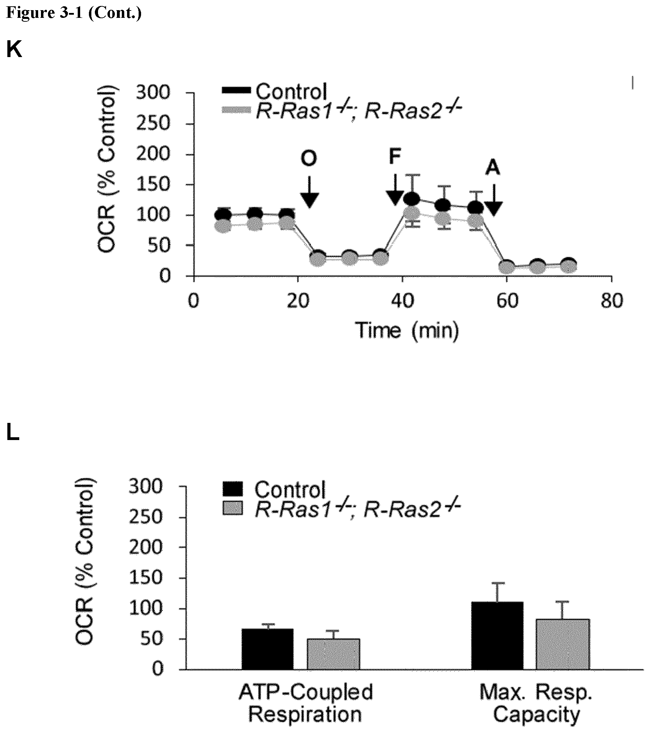

**Figure 3****. Myelin deficiency in *R-Ras1*^{-/-} mice leads to an increase in mitochondrial activity.** A, Polarographic profiles of isolated mitochondria from brain extracts of control and mutant adult mice (P60) in presence of 10 nM glutamate/malate, 0.5 mM ADP, 5 µM oligomycin (O), 5 µM FCCP (F) and 1 µM antimycin A (A), evidence higher mitochondrial activity in *R-Ras1*^{-/-} simple and double-mutants. ***B***, Polarographic profiles of isolated mitochondria from brain extracts of control and mutant newborn (P0) mice in presence of 10 nM glutamate/malate, 0.5 mM ADP, 5 µM oligomycin (O), 5 µM FCCP (F) and 1 µM antimycin A (A), didn't show any difference between the mitochondrial activity in simple and double-mutants compared to control. ***C***, Graph showing the quantification of Clark-type electrode results in adult (P60) mice mitochondria. State III respiration expressed in nmol O2/min/mg protein was: 18.3 ± 3.4 for control; 38.6 ± 7.4 for *R-Ras1*^{-/-}*,* 14.6 ± 2.5 for *R-Ras2*^{-/-} and 31.3 ± 3.1 for *R-Ras1*^{-/-}*;R-Ras2*^{-/-}*.* This data showed significant differences in *R-Ras1*^{-/-} (**p* < 0.05) and the double-mutant mice (**p* < 0.05) *(n* = 4). ***D***, Graph showing the quantification of Clark-type electrode results in newborn (P0) mice mitochondria. State III respiration expressed in nmol O2/min/mg protein was: 23.4 ± 1.4 for control, 23.9 ± 1.0 for *R-Ras1*^{-/-}*,* 20.6 ± 6 for *R-Ras2*^{-/-} and 18.8 ± 3.9 *R-Ras1*^{-/-}*;R-Ras2*^{-/-} . This data didn't show any significant differences (*n* = 4). ***E***, Representative scheme of the degree of myelination in adults vs in newborn mice, with a western blot of myelin (MBP) expression in total brain lysates of newborn (P0) and adult (P60) control mice. The total absence of myelin observed in the newborn samples indicates that at P0 is a non-myelinated system.
**Figure 3-1****. Purified oligodendrocytes and neurons from simple and double mutant mice do not display intrinsic mitochondrial alterations**. Seahorse assays developed on purified OLs and neurons of *R-Ras1*^{-/-}*, R-Ras2*^{-/-} and *R-Ras1*^{-/-}*;R-Ras2*^{-/-} have not shown mitochondrial intrinsic alterations. *A-F,* Measurements of Oxygen Consumption Rate (OCR) percentage over control, ATP-coupled respiration and maximal respiratory capacity in neurons purified cultures *in vitro,* have not shown any differences compared to control. For ATP-coupled respiration: control vs *R-Ras1*^{-/-} was 62.4 ± 9.8 in control, 66.2 ± 7.2 for *R-Ras1*^{-/-}; control vs *R-Ras2*^{-/-} was 57.6 ± 23.8 in control and 69.9 ± 11.7 for *R-Ras2*^{-/-}; and for Control vs *R-Ras1*^{-/-};*R-Ras2*^{-/-} was 64.7 ± 13.1 in control and 71.8 ± 10.3 for *R-Ras1*^{-/-}*;R-Ras2*^{-/-}*.* For maximal respiratory capacity: control vs *R-Ras1*^{-/-} was 203.7 ± 32.1 in control and 203.4 ± 19.2 for *R-Ras1*^{-/-}; control vs R-*Ras2*^{-/-} was 125.2 ± 34.8 in control and 117.9 ± 25.5 for *R-Ras2*^{-/-}; and for control vs *R-Ras1*^{-/-} ;*R-Ras2*^{-/-} was 96.3 ± 28.1 in control; 102.1 ± 17.4 for *R-Ras1*^{-/-}*;R-Ras2*^{-/-} *(n =* 3 independent cultures, with 6-12 wells per culture per genotype). ***G-L***, Measurements of Oxygen Consumption Rate (OCR) percentage over control, ATP-coupled respiration and maximal respiratory capacity in OLs purified cultures *in vitro,* have not shown any differences compared to control. Graphic showing the results of oligodendrocytes ATP-coupled respiration and maximal respiratory capacity, expressed in oxygen consumption rate (OCR) percentage over control. For ATP-coupled respiration: control vs *R-Ras1*^{-/-} was 69.7 ± 18.4 in control, 66.0 ± 19.5 for *R-Ras1*^{-/-}; control vs *R-Ras2*^{-/-} was 65.9 ± 7.1 in control, 67.2 ± 13.2 for *R-Ras2*^{-/-}; and for control vs R-*Ras1*^{-/-};*R-Ras2*^{-/-} was 66.5 ± 7.2 in control and 50.9 ± 12.26 for *R-Ras1*^{-/-}*;R-Ras2*^{-/-}*.* For maximal respiratory capacity: control vs *R-Ras1*^{-/-} was 105.75 ± 21.4 in control, 106.4 ± 14.2 for *R-Ras1*^{-/-}; control vs *R-Ras2*^{-/-} was 91.7 ± 27.4 in control, 122.4 ± 30.6 for *R-Ras2*^{-/-}; and for control vs *R-Ras1*^{-/-};*R-Ras2*^{-/-} was 110.0 ± 31.9 in control and 82.6 ± 28.5 for *R-Ras1*^{-/-}*;R-Ras2*^{-/-} *(n =* 3 independent cultures, with 6-12 wells per culture per genotype).

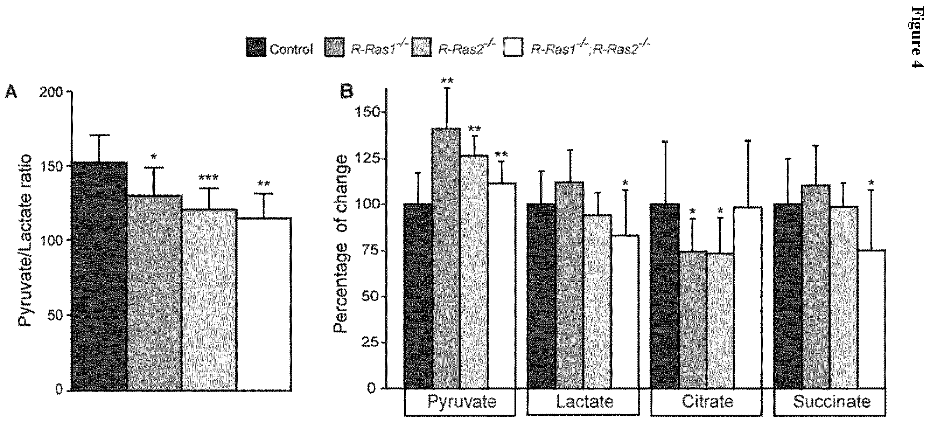

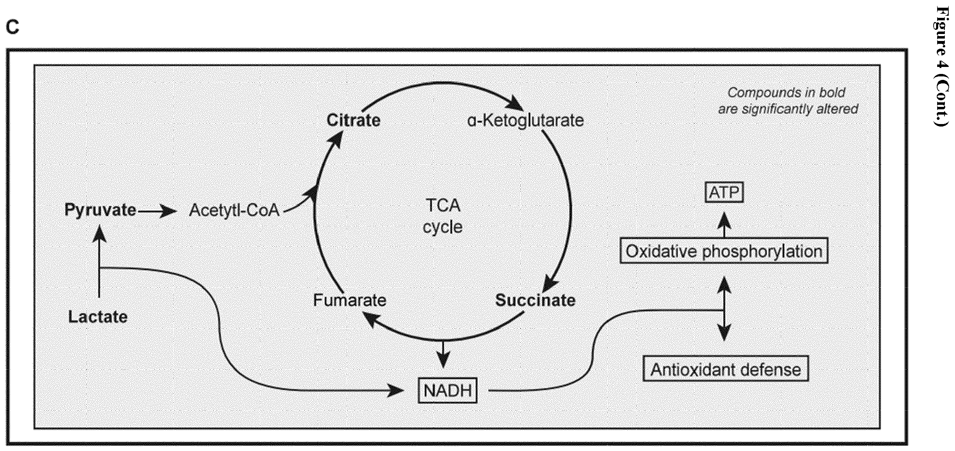

**Figure 4****. Absence of R-Ras1 and/or R-Ras2 modifies metabolism in hypomielinizated tracts. *A***, Lactate/Pyruvate ratio (x10³) is decreased in simple and double mutant mice compared to control. Bar graph represents mean ± SD. Results were: WT 153.53 ± 18.11, *R-Ras1*^{-/-} 131.21 ± 18.91 (**p <* 0.05), *R-Ras21*^{-/-} 121.75 ± 14.44 (****p <* 0.001) and *R-Ras1*^{-/-}*;R-Ras21*^{-/-} 116.19 ± 16.42 (***p <* 0.01) (*n* = 9). ***B***, Percentage of change of altered metabolites. Bar graph represents mean ± SD of the percentage of change relative to the control. Results for *R-Ras1*^{-/-} were: pyruvate (+47.6%; ***p <* 0.01); citrate (-25.6%; **p <* 0.05); for *R-Ras2*^{-/-}: pyruvate (+26.6%; ***p* < 0.01); citrate (-27.0%; **p* < 0.05); and for *R-Ras1*^{-/-}*;R-Ras2*^{-/-}: pyruvate (+11.6%; ***p* < 0.01); lactate (-17.0%; **p* < 0.05); succinate (-25.18%; **p <* 0.05) (*n =* 9). ***C***, Schematic representation showing the possible link between the detected altered metabolites and their fate in support of ATP production and antioxidant defense. Optic nerve samples were obtained from adult mice (P90).

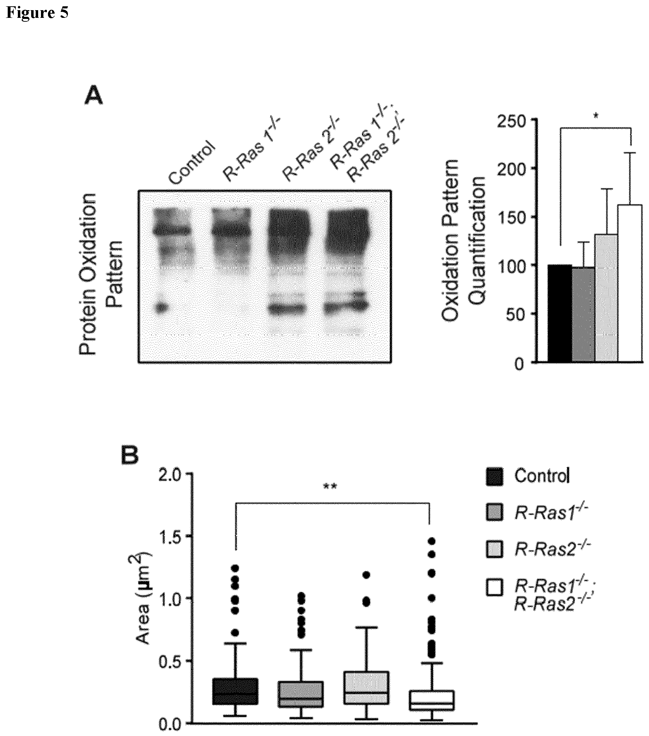

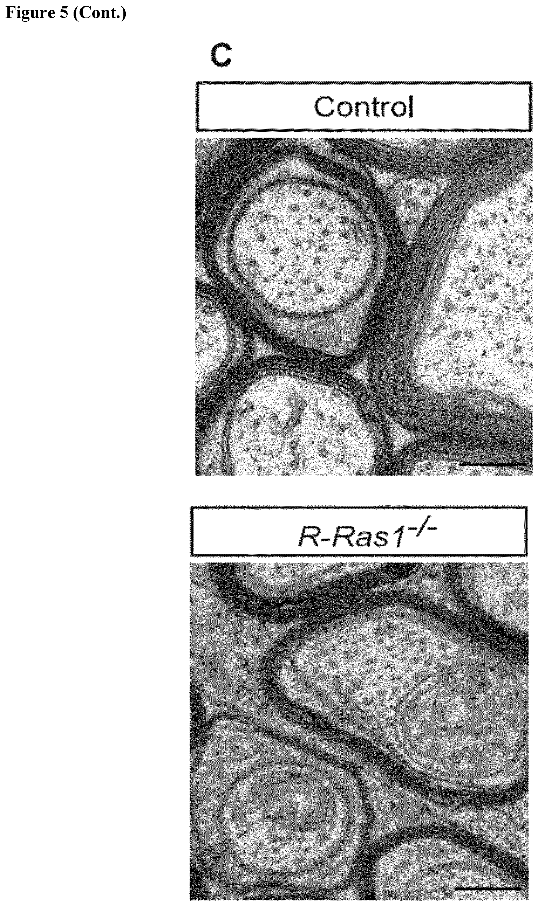

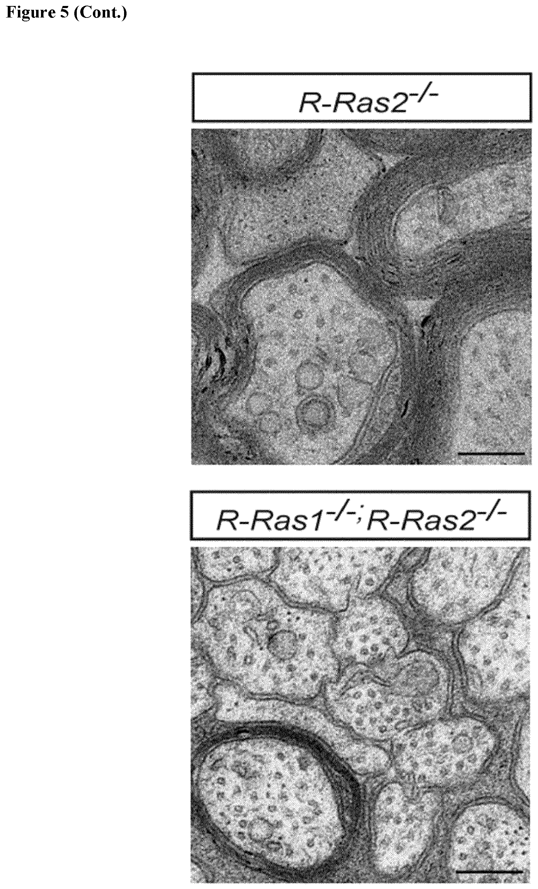

**Figure 5****. High levels of protein oxidation and alterations in axonal area in *R-Ras1*^{-/-}*;R-Ras2*^{-/-} mutant mice.** ***A**,* Western blot of the protein oxidation pattern of optic nerve lysates from single and double R-Ras mutant adult mice (P90) compared with controls, showing an increase in the oxidation in the absence of R-Ras2. Significant increase in Protein Oxidation Pattern in double mutants compared with controls. Bar graph represents mean ± SD of the fold change relative to the control. *R-Ras1*^{-/-} 97.7 ± 26.5%, *R-Ras2*^{-/-} 131.8 ± 46.6%, *R-Ras1*^{-/-}*;R-Ras2*^{-/-} 162.4 ± 53.4% (**p <* 0.05) *(n =* 5). ***B***, Axonal area presented in the optic nerve of control, R-*Ras1*^{-/-}*, R-Ras2*^{-/-} and *R-Ras1*^{-/-}*;R-Ras2*^{-/-} mice. Significant differences were observed in *R-Ras1*^{-/-} *;R-Ras2*^{-/-} mice compared to the control. Control 0.30 ± 0.22 µm (*n =* 131 axons analyzed), R-*Ras1*^{-/-} 0.26 ± 0.19 µm (*n* = 145 axons analyzed), *R-Ras2*^{-/-} 0.32 ± 0.24 µm (*n* = 81 axons analyzed), *R-Ras1*^{-/-}*;R-Ras2*^{-/-}0.23 ± 0.22 µm (*n* = 219 axons analyzed) (***p <* 0.01) (*n =* 3 mice per genotype). ***C***, Transverse sections of the optic nerve from adult (P90) control, *R-Ras1*^{-/-} *R-Ras2*^{-/-} and *R-Ras1*^{-/-}*;R-Ras2*^{-/-} mice analyzed by electron microscopy have shown a decrease of axon area in double-mutant mice compared to control. Scale bar, 200 nm.

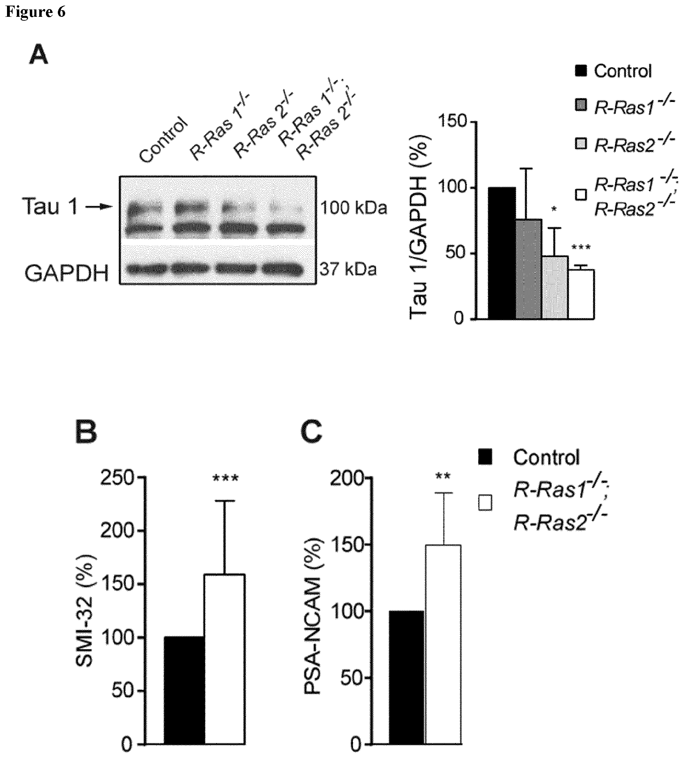

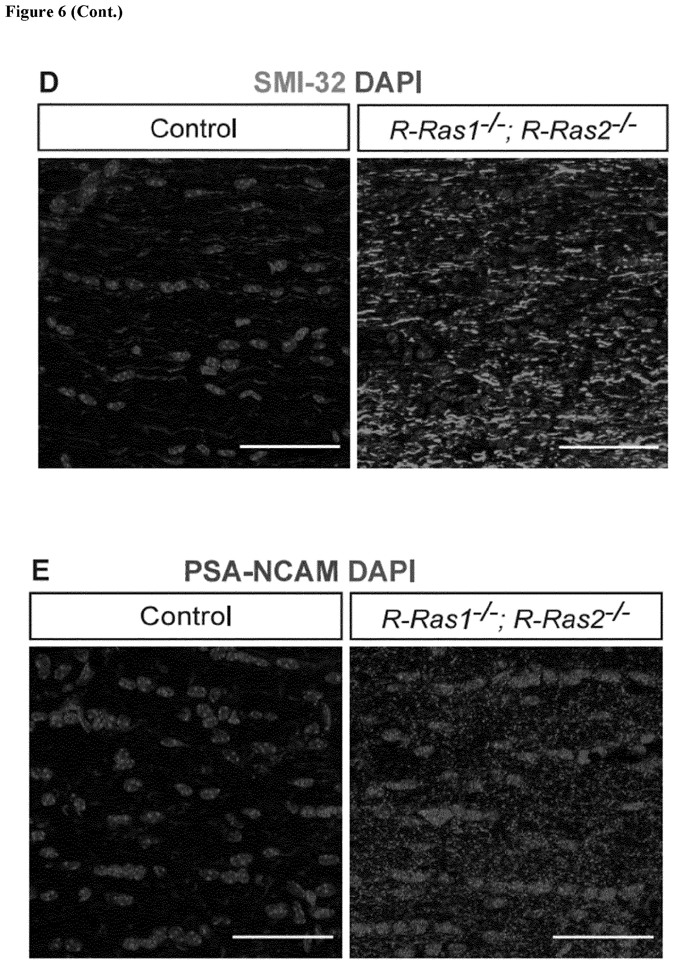

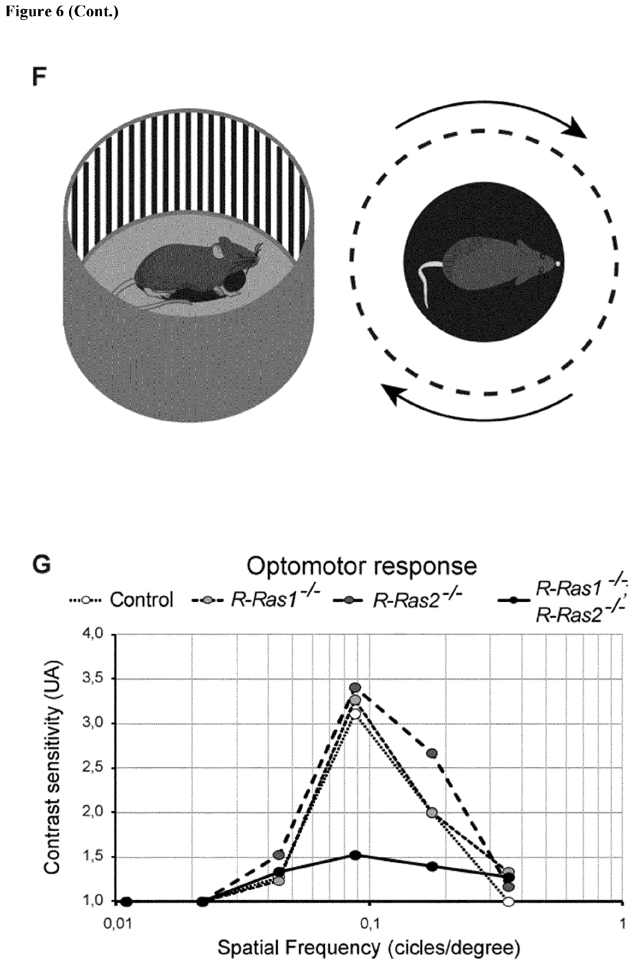

**Figure 6****. Absence of R-Ras1 and R-Ras2 causes loss of integrity in axonal cytoskeleton components associated with degeneration and the loss of function in the optic nerves of *R-Ras1*^{-/-}*,R-Ras2*^{-/-} mice.** ***A**,* Western blot of Tau 1 in optic nerve lysates from adult mice (P90), showing a reduction of the Tau 1 phosphorylated isoform in *R-Ras1*^{-/-}, *R-Ras2*^{-/-} and *R-Ras1*^{-/-}*;R-Ras2*^{-/-} compared with controls. Bar graph represents mean ± SD of the change relative to the control of measurements (normalized to GAPDH) from three different experiments: *R-Ras1*^{-/-} 75.9 ± 38.5%, *R-Ras2*^{-/-} 47.7 ± 22.1% (**p* < 0.05), *R-Ras1*^{-/-}*;R-Ras2*^{-/-} 37.4 ± 3.82% (****p* < 0.0001). ***B***, Graph of SMI-32 Mean Fluorescence Quantification showing a significant increase in double mutant mice compared to controls. Bar graph represents mean ± SD of the change relative to the control: *R-Ras1*^{-/-}*;R-Ras2*^{-/-} 158.7 ± 69% (****p* < 0.0001) (WT: *n* = 32 pictures analyzed, *R-Ras1*^{-/-}*;R-Ras2*^{-/-}: *n* = 29 pictures analyzed). ***C***, Graph of PSA-NCAM Mean Fluorescence Quantification showing a significant increase in double mutant mice compared to controls. Bar graph represents mean ± SD of the change relative to the control: *R-Ras1*^{-/-}*;R-Ras2*^{-/-} 150 ± 38.9% (***p* < 0.01) (WT: *n =* 14 pictures analyzed, *R-Ras1*^{-/-}*;R-Ras2*^{-/-}: *n* = 18 pictures analyzed). ***D***, Confocal microscopy images of longitudinal sections of optic nerves from adult mice (P90) immunostained with SMI-32 revealed an increase of SMI-32 staining in *R-Ras1*^{-/-}*;R-Ras2* ^{-/-} mice relative to controls. ***E***, Confocal Microscopy images of longitudinal sections of optic nerves from adult (P90) immunostained with PSA-NCAM revealed an increase of PSA-NCAM staining in *R-Ras1*^{-/-}*;R-Ras2*^{-/-} mice relative to controls. ***F***, Schematic representation of homemade optomotor device where Optomotor test was performed. ***G***, Optokinetic responses of control, *R-Ras1*^{-/-}*, R-Ras2*^{-/-} and *R-Ras1*^{-/-}*;R-Ras2*^{-/-} mice. Contrast sensitivity is represented as a function of spatial frequency as the mean ± SD. The control group included WT mice (*n* = 8), and the mutant group included *R-Ras1*^{-/-}*, R-Ras2*^{-/-} and *R-Ras1*^{*-*/}*⁻;R-Ras2*^{-/-} mice (each group *n* = 8). Statistical differences between the double mutant and control mice (****p* < 0.001, two-way ANOVA) were found for intermediate spatial frequencies: 0.088, and 0.177 cycles/degree. No significant statistical differences were found between single mutant and control animals. ***A-C**,* All samples were obtained from adult mice (P90) (*n* = 3). Scale bar, 50 µm.

### Detailed description of the invention

The present invention is illustrated by means of the examples below which should not be interpreted a limitation in the scope of protection offered by the present invention.

### Example 1. MATERIAL AND METHODS.

### Example 1.1. Animals.

C57BL6 Mice were housed in specific pathogen-free conditions in a humidity- and temperature-controlled room on a 12 h light/dark cycle, receiving water and food *ad libitum.* All experiments were performed in male and female mice. All animal procedures were approved by the corresponding institutional ethical committee (Centro de Biologia Molecular Severo Ochoa, CBMSO) and were performed in accordance with Spanish and European directives. All efforts were made to minimize animal suffering.
*R-Ras1*^{-/-} mice were generated using the targeting construction BAL1-HR with a neomycin resistance cassette flanked by FRT sequences inserted in intron 1 and LoxP sites flanking exons 2 and 6. The construction was electroporated into embryonic stem cells derived from mouse 129Sv/Pas and selected by G418 antibiotic. Southern blot was used to verify the correct homologous recombination.
Heterozygous mice were crossed, and offspring littermates were genotyped by PCR (*R-Ras1*^{-/-} FW: 5'- *SEQ ID NO: 1* -3', *R-Ras1*^{-/-} RV: 5'- *SEQ ID NO:* 2 -3', *R-Ras1*^{+/+} FW: 5'- *SEQ ID NO:* 3 -3', *R-Ras1*^{+/+} RV: 5'- SEQ ID NO: 1-3').
*R-Ras2*^{-/-} mice were derived from embryonic stem cell clone OST361011 with insertion of retroviral VICTR37 in the middle of intron 1 of *R-Ras2.* Heterozygous mice were crossed, and offspring littermates were genotyped by PCR (primer 1, 5'- *SEQ ID NO:* 4 -3'; primer 2, 5'- *SEQ ID NO: 5* -3'; primer 3, 5'- *SEQ ID NO: 6* -3'). *R-Ras* 2^{+/+} and *R-Ras2*^{-/-} transgenic mice were obtained by crossing heterozygous mice.
Double-knock-out *R-Ras1*^{-/-}*;R-Ras2*^{-/-} mice were generated by backcrossing individual lines R-*Ras1*^{-/-} and *R-Ras2*^{-/-}*.* Animals were maintained in a C57BL6J background. We used either male and female mice to perform the experiments. We defined newborn as postnatal 0 (P0).

### Example 1.2. Western blotting.

Tissue samples (optic nerve, cerebral cortex and total brain of animals from P0 to P90) were dissected, resuspended in lysis buffer (50 mM Tris pH 8.0, 150 mM NaCl, 1% NP40, 2mM EDTA, 0.1% SDS, 0.5% deoxycholate, and protease inhibition mixture; Roche 11697498001) and phenylmethane sulfonyl fluoride (PMSF). Lysates were denatured boiling them for 5 min in protein loading buffer [50 mM Tris-HCl pH 6.8, 2% SDS, 10% glycerol, 1% β-mercaptoethanol (BME), 12.5 mM EDTA and 0.02% bromophenol blue] and resolved in 10-12% SDSP-gels in the presence of BME. Gels were run at constant current starting at 90 or 100V. After electrophoresis, samples were transferred by electroblotting onto a PVDF membrane in a semidry electroblotting system (Transblot-turbo. Bio Rad laboratories; California, USA) at 1.2 mA/cm² for 35-40 min. Nonspecific protein binding was blocked by incubating the membrane with 5% non-fat milk in TBS-Tween-20 for 1 h at room temperature. Membranes were incubated overnight with the pertinent primary antibodies in blocking buffer: mouse anti-ATP synthase (subunit *α*) 1:1000 (Invitrogen catalog# 459240), mouse anti-UQCRC2 (Ubiquinol-cytochrome c reductase complex) 1:3000 (Thermo Fisher Scientific catalog# PA5-30204), mouse anti-CoxIV (cytochrome oxidase subunit IV) 1:1000 (Molecular Probes catalog# A-21348,). Other antibodies used were mouse anti-GAPDH (G9) 1:1000 (Santa Cruz Biotechnology catalog# sc-365062, RRID:AB_10847862), mouse anti-MBP (aa67-74) 1:200 (Bio-Rad / AbD Serotec catalog# MCA685S, RRID:AB_325009) and mouse anti-Tau 1 1:1000 that was kindly provided by Prof. J. Ávila (CBMSO, Madrid). After washing, blots were incubated for 1 h with appropriated peroxidase-conjugated secondary antibodies (Thermo Fisher Scientific). Labeled proteins were detected with the Chemiluminescence Reagent ECL (GE Healthcare).

### Example 1.3. Oxyblot® (OxyBlot Protein Oxidation Detection Kit®).

OxyBlot assay was performed according to manufacturer's specifications (Sigma-Aldrich catalog# S7150). It consists on the detection of the carbonyl groups that are introduced into the amino acid side chain after oxidative modification of proteins previously derivatizated to a 2, 4-dinitrophenyl-hydrazone (DNP-hydrazone). Then DNP-derivatized proteins were resolved in 12% SDSP-gels samples and transferred by electroblotting onto a PVDF membrane (previously activated in EtOH 95%) in a semidry electroblotting system (Transblot-turbo, Bio Rad laboratories) at 1.2 mA/cm² for 35-40 min. Nonspecific protein binding was blocked by incubating the membrane with 5% non-fat milk in TBS-Tween-20 for 1h at room temperature. Then membranes were exposed to a primary rabbit anti-DNPH protein antibody 1:100 for 1 h, and then to a secondary goat anti-rabbit IgG (HRP-conjugated) antibody 1:300 for 1 h at room temperature (both antibodies were diluted in TBS-Tween-20). After incubations, membranes were washed gently with TBS-Tween-20. Finally, the detection was performed with Chemiluminescence Reagent ECL (GE Healthcare).

### Example 1.3. Immunohistochemistry.

Animals were anesthetized (ketamine/xylazine) and perfused transcardially with 0.1 M phosphate-buffered saline (PBS; pH 7.4) followed by 4% paraformaldehyde in PBS. Perfused tissues were removed and postfixed in 4% paraformaldehyde at 4°C overnight. Then they were cryoprotected in 30% sucrose in PBS and embedded and frozen in a 7.5% gelatin in 15% sucrose solution. Then they were sectioned on a cryostat to produce 20 µm cryosections on Superfrost Plus microscope slides (Thermo Fisher Scientific). Sections were blocked for 1h at room temperature with 10% fetal bovine serum in PBS containing 0.5% Triton-X 100 (blocking solution) and then incubated overnight at 4°C with the primary antibodies mouse anti-SMI-32 1:1000 (Biolegend catalog# 801701, RRID:AB_2564642) or mouse anti-PSA-NCAM 1:50 (Miltenyi Biotec 130-117-394, RRID:AB_2727931) diluted in blocking solution. After 3 washes, fluorescent-tagged secondary antibodies (Alexa-488 or Alexa- 647) were applied for 1 h at room temperature, and sections were counterstained with DAPI (Sigma-Aldrich catalog# 32670) and mounted in Aqua-polymount mounting medium (Polyscience catalog# 18606).

### Example 1.4. Confocal microscopy.

Fluorescence images were obtained using a confocal multispectral Leica TCS SP5 system (Leica Microsystems) controlled by Las AF v 2.7 software (Leica). Image acquisition was performed sequentially using a 40x/1.4NA oil immersion objectives and appropriate fluorochrome excitation lines (405 nm, 488 nm, and 647 nm for DAPI, Alexa-488 and Alexa-647, respectively). Mean intensity was quantified using Fiji/ImageJ software.

### Example 1.5. Electron microscopy.

Mutant and littermate control mice were anesthetized as indicated above, intracardially perfused with 4 % PFA and 2.5 % glutaraldehyde in 0.1M PBS and treated in the same fixative overnight. Optic nerves were then removed after several washes in PBS and the sections were postfixed with 1 % osmium tetroxide in double-distillated water and 1% potassium ferrocyanide 1 h at 4°C. After 3 washes with double-distillated water, they were treated with 0.15% of tanic acid in 0.1M PBS, pH 7.4 and block-stained with 2 % uranyl acetate in distilled water for 1 h room temperature in darkness. Sections were then washed 3 times with double-distilled water and dehydrated in an ascending series of ethanol dilutions up to 100 % at 4°C. The following infiltration was made with propylene oxide:EtOH (1:1; v:v) for 5 min, propylene oxide 3 times 15 min each, propylene oxide:Epon (1:1) (epoxy resin TAAB 812, TAAB laboratories) for 45 minutes, 100% Epon 1h and Epon 100% overnight. Encapsulation was made in flat molds where optic nerves were correctly orientated and then polymerized 48 h at 60°C. The optic nerve was cut in 70-80 nm sections on an ultramicrotome (Leica Ultracut UCT) with a diamond blade (Diatome) and collected on Cu-Pd boutonniere grids covered by Formvar. Staining of ultrathin sections staining was performed by drops of 2% aqueous uranyl acetate for 7 min, followed by Reynolds's lead citrate for 2 min. Ultrastructural analyses were performed with a JEM-1010 electron microscope (Jeol). 12 photographs were taken along the section covering the whole diameter of the optic nerve using a CMOS 4K x 4K, F416 de TVIPS camera (Gauting). From these pictures, six in perfect condition that matched the mutants and control. These photographs were used for quantifying the number of mitochondria in myelinated and non-myelinated axons. We analyzed 800-1000 axons per genotype. It was also measured the axons area (around 125 axons per genotype). Three animals per genotype were analyzed. Identical results were obtained independently four times by different investigators. Images were processed using Fiji/ImageJ software.

### Example 1.6. Oxygen consumption rates (OCR).

Mitochondrial OCR was measured by a Clark-type electrode as described previously (Formentini et al., 2014). Briefly, Adult (P90) and newborn (P0) isolated brain mitochondria (200 µg protein) were prepared in buffer A (320 mM sucrose, 1 mM EDTA, and 10 mM Tris-HCl, pH 7.4) after the permeabilization of synaptosomes with 100 µM digitonine. Samples Oxygen consumption rate (OCR) was determined using glutamate plus malate (10mM) as respiratory substrate in presence and absence of 0.5 mM ADP, 5 µM oligomycin (OL), 5 µM FCCP and 1 µM antimicyn A. The composition of the respiration buffer is 225 mM sucrose, 5 mM MgCl2, 10 mM KCl, 10 mM phosphate buffer, 1 mM EGTA, 0.05% BSA and 10 mM Tris-HCl, pH 7.4.

### Example 1.7. Extracellular Flux Analysis ("Seahorse assay").

Seahorse assay on neurons and glial cells was performed as described previously (Katsu-Jiménez and Giménez-Cassina, 2019). Oxygen consumption rate (OCR), ATP-Coupled Respiration and Maximal Respiration Capacity were determined in real time using XF24 extracellular flux analyzer (Seahorse Bioscience-Agilent, Santa Clara CA). Purified and independent neurons and OLs cultures were seeded at 1.5x10⁵ or 4x10⁴ cells per well, respectively, in specific growth medium and then analyzed using the Seahorse XF24 Extracellular Flux Analyzer. One hour prior to the experiment, culture medium was replaced by assay medium consisting on bicarbonate-free DMEM supplemented with 5 mM glucose and 1 mM sodium pyruvate. After baseline measurement, the following sequential injections were made to determine ATP-coupled respiration and maximal respiratory capacity: 1 µM oligomycin, 0.5 µM carbonyl cyanide-4 (trifluoromethoxy) phenylhydrazone (FCCP), and 4 µM rotenone/antimycin A.

### Example 1.8. Metabolomics.

The Metabolomics assay was performed at CEMBIO (Madrid, Spain), as described previously at (Gonzalez-Riano et al., 2018). Briefly, the Gas Chromatography-Mass Spectrometry (GC-MS) analysis was developed with optic nerve samples from control, simple, and double-mutant mice. Optic Nerves were dissected from adult mice (P90) after cervical dislocation. Samples were then kept at - 80°C immediately until the day of the analysis. First, 100 µl of cold methanol (-20°C) containing 25 ppm of pentadecanoid acid used as an Internal Standard (IS) was added to each sample. Subsequently, samples were homogenized using a 2 mm particle size glass beads from TissueLyser LT homogenizer (Qiagen, Germany), centrifuged at 13.000g at 4°C for 10 min, and 80 µl of the supernatant from each sample was transferred to GC-MS vial. Supernatants were evaporated until dryness using a SpeedVac Concentrator System. Later, samples were chemically derivatized with two reactions methoxymation and then silylation for GC-MS analysis. Before the analysis, 100 µL of heptane containing 10 ppm of methyl stearate C18:0 as a second IS was added. Finally, 2 µL of derivatized sample was injected in a split mode (1:10) into a GC instrument 7890A (Agilent Technologies, Germany) coupled to an inert mass spectrometer with triple-Axis detector 5975C (Agilent Technologies, Germany). The quality and the robustness of the analytical run was evaluated using quality control samples prepared by pooling equal volumes of homogenate from each sample. Raw data files were imported into Agilent Mass Hunter Unknown Analysis Tool 7.0 for peak detection, deconvolution, and metabolite identification. The software executed a search against two targeted libraries to obtain a chemical identity for the compounds: Fiehn version 2013 and the CEMBIO internal spectrum library, always comparing both the retention time (RT) and the spectra extracted during deconvolution against each compound included in the library. A third commercial library-NIST (National Institute of Standards and Technology, library 2.2 version 2017) - was used for unknown identification. Those metabolites with a spectrum score of ≥ 80% and a concordant retention index based on the *n*-alkane scale were tentatively identified according to this library. Data were filtered by the signal variation coefficient (CV) in QC, considering acceptable values below 30%. Finally, altered metabolic pathways were analyzed using MetaboAnalyst® 4.0.

### Example 1.9. Optomotor response.

Optomotor test was performed as described previously (Prusky et al., 2004). This test was proposed as a test of adequate animal behavior to evaluate the functional state of the retina's visual function in mice that suffer from retinal degenerations. Experimental animals respond to visualized rotating vertical bars by characteristic reflex movement of their head in the same direction of bars rotation. Contrast sensitivity is actually used as an evidence of visual discrimination (Umino et al., 2008). A homemade optomotor device was built based on (Prusky et al., 2004). Mice were placed in the center of a square array of computer monitors that displayed stimulus gratings. Mice were monitored by the use of infrared television camera placed on top of the testing chamber. The visual test consisted in vertical black/white bars (gratings) of distinct spatial frequencies (0.011 to 0.355 cycles/degree) moving through the screens. Contrast sensitivity was calculated as the inverse of contrast threshold between white and black bars. Maximum contrast (value = 1) was measured for white screen luminance of 122,70 cd/m² and black screen luminance of 0.25 cd/m²). The Vision Egg tool was used for light stimulation. The test started with the easiest stimulus to mice, with a spatial frequency of 0.088 cycles/degree, a temporal frequency of 0.88 Hz and a normalized contrast of 1.

### Example 1.10. Statistical Analysis.

Quantitative data are shown as the mean ± standard deviation (SD). The experimental groups were compared using a two-tailed Student's t-test. Statistical numeric data are provided in the figure legends. (*) means *p* < 0.05; (**) means *p* < 0.01; (***) means *p* < 0.001. For Metabolomics Statistical Analysis we used Benjamini and Hochberg and Mann-Whitney tests. An alpha level of 0.05 was considered significant. Outliers were identified using boxplots. These outliers were discarded as being outside the median trend. For Optomotor response Statistical Analysis we used a two-way ANOVA test and Bonferroni post-tests.

### Example 2. RESULTS.

### Example 2.1. Absence of R-Ras2 leads to increased numbers of axonal mitochondria.

Here, we set out to investigate the metabolic adaptation and neuronal dysfunction resulting from R-Ras loss of function as mechanisms mimicking aspects of myelinating disorders. We first set out to perform additional electron microscopy analysis of optic nerve cross sections to quantify relationships between myelination defects, mitochondrial phenotype and R-Ras genotypes. Consistent with our prior data, visual inspection of sections from *R-Ras1*^{-/-} mice (*R-Ras1* KO mice) showed thinner myelin layers whereas sections from *R-Ras2*^{-/-} mice *(R-Ras2* KO mice) had fewer myelinated axons relative to sections from wildtype (WT) mice. Consolidating these phenotypes, sections from *R-Ras1*^{-/-}*;R-Ras2*^{-/-} (DKO) mice showed an overall more severe myelin phenotype. Whereas roughly less than 30% of axons in WT mice were non-myelinated, the relative proportion of non-myelinated to total axons increased dramatically in DKO mice (82.76 ± 8.81% vs 27.87 ± 9.82%, *p* < 0.001) (**Fig. 1*****B***). The ratio of non-myelinated axons to total axons in WT mice was comparable to *R-Ras1* KO mice (26.34 ± 9.13%) but increased in *R-Ras2* KO mice (47.98 ± 17.24%; *p <* 0.01) (**Fig. 1*****B***), indicating an additive effect from loss of both R-Ras1 and R-Ras2 in the DKO.

We found that axonal numbers of mitochondria were increased in *R-Ras2* KO and DKO mice (mitochondria per field: 7.37± 0.98, *p* < 0.001 and 10.25 ± 1.68, *p* < 0.001) (**Fig. 1*****C***). Similar to the quantification of demyelinated axons, mitochondrial numbers were comparable between WT and *R-Ras1* KO (mitochondria per field: 5.81 ± 1.12 and 5.83 ± 1.11) (**Fig. 1*****C***). Calculated as percentages, these data reflect that mitochondrial density was increased in *R-Ras2* KO mice (26.83 ± 16.88%) and substantially more so in DKO mice (76.38 ± 28.92%) (data not shown). The *R-Ras1* KO neurons did not show differences in mitochondria numbers compared to control.

To determine whether increase in mitochondrial density segregated with loss of axonal myelination, we next quantified the number of mitochondria present in myelinated and non-myelinated axons. We found that myelinated axons in mice across control and all *R-Ras* genotypes contained roughly equal numbers of mitochondria (**Fig. 1*****D***, mitochondria/field WT: 5.44 ± 0.96, *R-Ras1* KO: 5.59 ± 1.17, *R-Ras2* KO: 5.32 ± 0.36 for *R-Ras2*^{-*l*-}*,* and *R-Ras1*/*2* DKO mice: 4.55 ± 2.72). In contrast, axons lacking a myelin sheath contained significantly more mitochondria in both *R-Ras2* KO and DKO mice (mitochondria per field: 2.05 ± 0.64, *p* < 0.001 and 5.71 ± 2.67, *p* < 0.001) relative to WT and *R-Ras1* KO (mitochondria per field: 0.37 ± 0.18 and 0.24 ± 0.14) (**Fig. 1*****E***). To ascertain how absence of myelin segregates with numbers of axonal mitochondria, we normalized the number of mitochondria relative to the number of non-myelinated axons in WT and mutant mice. This ratio revealed that a complete loss of myelin increases the number of mitochondria in axons lacking R-Ras2 to levels similar to DKO mice (mitochondria per field: 0.62 ± 0.20, *p* < 0.001 vs 0.67 ± 0.23, *p <* 0.001) whereas the normalized mitochondrial number count was comparable in WT and *R-Ras1* KO mice (mitochondria per field: 0.22 ± 0.09 vs 0.13 ± 0.09) (**Fig**. **1*****F***).

These data indicate that a complete loss of the myelin sheath leads to a metabolic adaptation reflected as an increase in numbers of axonal mitochondria, whereas a mere decrease in thickness of the myelin sheath surrounding axons appears to not be sufficient to impact on the number of mitochondria (**Fig. 1*****G***).

### Example 2.2. Absence of R-Ras1 increases protein expression of mitochondrial complexes.

We next asked whether R-Ras loss of function genotypes resulted in changes in mitochondrial activity. To answer this question, we quantified expression of proteins executing mitochondrial oxidative phosphorylation (OXPHOS) in optic nerve homogenates in adult mutant mice and WT controls. In OXPHOS, a group of complexes in the inner mitochondrial membrane create ATP by coordinating serial redox reactions to free up the energy captured in the double bond of O². With western blot analysis, we found that expression of complex III (cytochrome c reductase), complex IV (cytochrome c oxidase), and/or complex V (ATP synthase) subunits were increased in *R-Ras1* KO and DKO mice (**Fig. *2A***)*.* We did not detect any significant differences between *R-Ras2* KO homogenates and controls. The fold-change in expression values for the different mitochondrial complexes relative to control were as follows: Complex V (ATP synthase): R-*Ras1* KO = 1.97 ± 0.09 (*p <* 0.001); *R-Ras2* KO = 1.37 ± 0.76; DKO = 2.08 ± 0.84 (*p* < 0.05). Complex IV (COX IV): *R-Ras1* KO = 2.45 ± 0.45 (*p* < 0.001); *R-Ras2* KO = 1.13 ± 0.36; DKO = 2.67 ± 0.21 (*p <* 0.001). Complex III (UQCRC2): *R-Ras1* KO = 0.91 ± 0.29; *R-Ras2* KO = 0.93 ± 0.70; DKO = 3.40 ± 0.92 (*p* < 0.001) (**Fig. 2*****A***)*.* To ascertain if the observed increases in expression of OXPHOS components were specific to myelin-dependent tracts, such as the optic nerve, we repeated the western blot analysis in homogenates from cerebral cortex, a brain region which contain low levels of myelin. The expression levels in cerebral cortex for Complex V (ATP synthase) were: *R-Ras1* KO = 1.22 ± 0.02 increase; *R-Ras2* KO = 1.18 ± 0.15; DKO = 1.24 ± 0.36, for Complex IV (COX IV): *R-Ras1* KO = 0.91 ± 0.04; *R-Ras2* KO = 0.91 ± 0.02; DKO = 0.87 ± 0.21, and for Complex III (UQCRC2): *R-Ras1 KO* = 1.02 ± 0.07; *R-Ras2 KO =* 1.05 ± 0.22; DKO = 1.13 ± 0.34 (**Fig. 2*****B***). We did not find any differences in the expression levels of ATP synthase, UQCRC2, or COXIV in mutant mice between myelin-dependent versus less-dependent myelin tissues (**Fig. 2*****B***). These results were confirmed in retinal homogenates, a non-dependent myelin tissue (data not shown).

Taken together, these data show that loss of R-Ras1 leads to increased expression of mitochondrial complexes UQCRC2, COXIV and ATP synthase only in myelin-dependent tracts, without causing alterations in weakly myelinated brain regions.

### Example 2.3. Loss of R-Ras1 increases oxygen consumption in adult brain.

To understand whether R-Ras1 and R-Ras2 loss leads to functional changes in mitochondrial activity, we measured oxygen consumption rates (OCR) in purified mitochondria isolated from adult brain using a Clark-type electrode (**Fig. 3*****A***). We did not observe quantifiable changes in State IV and State III respiration across genotypes (**Fig. *3A******,C***), however we found that ADP-stimulated State III respiration (expressed as nmol O₂ / min / mg protein) was significantly elevated in *R-Ras1* KO (38.6 ± 7.4, *p* < 0.05) and DKO mitochondria (31.3 ± 3.1, *p <* 0.05) relative to WT and *R-Ras2* KO (18.3 ± 3.4 and 14.6 ± 2.5) (**Fig. 3*****C***).

We next asked whether the increase in OXPHOS activity in *R-Ras1* KO and DKO mice was dependent on the presence of myelin. To this end, we analyzed the OCR in a non-myelinated tissue (**Fig. 3*****B***), specifically, in mitochondria isolated from neonatal P0 mouse brain (**Fig. 3*****E***) but we did not find any alterations in mitochondrial function in neonatal R-Ras mutant mice. State III respiration (nmol O₂ / min / mg protein) was: 23.4 ± 1.4 for WT; 23.9 ± 1.0 for *R-Ras1* KO 20.6 ± 6 for *R-Ras2* KO; and 18.8 ± 3.9 for DKO mice (**Fig. 3*****D***). Taken together, these data show that myelin deficiency in adult mice resulting from loss of R-Ras1 leads to a compensatory increase in mitochondrial activity, with no observed changes in mitochondrial respiration in non-myelin-dependent tissues.

### Example 2.4. Absence of R-Ras1 and/or R-Ras2 does not affect mitochondrial activity in OLs and isolated neurons.

To assess possible cell-intrinsic mitochondrial alterations in Ras mutant mice, we next compared mitochondrial activity of purified cultures of OLs and neurons isolated from R-Ras mutant and WT mice. To this end, we performed Seahorse assays to measure OCR in adherent cultures but did not detect any differences between mutant and control mice in either cell type **(****Fig. 3-1 A****,** **C** **and** **E**) and OLs (**Fig. 3-1 G****,** **I** **and** **K**). These results prove absence of cell-intrinsic mitochondrial alterations across neurons and OLs, suggesting that R-Ras1 and R-Ras2 are essential, from a mitochondrial perspective, for correct energetic coupling between the oligodendrocyte and the neuron it wraps around. Results of ATP-coupled respiration in cultured neurons: WT (62.4 ± 9.8 OCR) vs *R-Ras1* KO (66.2 ± 7.2 OCR); WT (57.6 ± 23.8 OCR) vs R-*Ras2* KO (69.9 ± 11.7 OCR); and WT (64.7 ± 13.1 OCR) vs the double knockout (71.8 ± 10.3 OCR) (**Fig. 3-1 *B, D*** **and** **F**). Results of maximal respiratory capacity in cultured neurons: WT (203.7 ± 32.1 OCR) vs *R-Ras1* KO (203.4 ± 19.2 OCR); WT (125.2 ± 34.8 OCR) vs *R-Ras2* KO (117.9 ± 25.5 OCR); and WT (96.3 ± 28.1 OCR) vs the double knockout (102.1 ± 17.4 OCR) (**Fig. 3-1 *B, D*** **and** **F**). In the case of ATP-coupled respiration in OLs: WT (69.7 ± 18.4 OCR) vs *R-Ras1* KO (66.0 ± 19.5 OCR); WT (65.9 ± 7.1 OCR) vs *R-Ras2* KO (67.2 ± 13.2 OCR); and WT (66.5 ± 7.2 OCR) vs the double knockout (50.9 ± 12.26 OCR) (**Fig. 3-1 *H******,*** ***J* and *L***)*.* Results of maximal respiratory capacity in OLs: WT (105.75 ± 21.4 OCR) vs *R-Ras1* KO (106.4 ± 14.2 OCR); WT (91.7 ± 27.4 OCR) vs *R-Ras2* KO (122.4 ± 30.6 OCR); and WT (110.0 ± 31.9 OCR) vs the double knockout (82.6 ± 28.5 OCR) (**Fig. 3-1 *H******,*** ***J*** **and** ***L***)*.* These results proved the absence of intrinsic mitochondrial alterations within neurons and OLs independently, suggesting that R-Ras1 and R-Ras2 are essential, from a mitochondrial perspective, for correct energetic coupling between the oligodendrocyte and neuron.

### Example 2.5. Absence of R-Ras1 and/or R-Ras2 alters metabolism in myelin deficient tissues.

We next asked in what way metabolic states of axons are affected by an increase in number and/or activity of mitochondria. To answer this question, we conducted an untargeted metabolomics-based study in the optic nerves of adult mice lacking R-Ras1 and/or R-Ras2, where we identified changes in concentrations of multiple metabolites with gas chromatography-mass spectrometry (GC-MS).

Lactate and pyruvate are considered indirect biomarkers of mitochondrial function, where increased lactate to pyruvate ratios can be a sign of mitochondrial dysfunction or mitochondrial disease. In R-Ras mutant mice, we found that lactate/pyruvate ratios were decreased in all Ras genotypes, but the most decreased in the DKO mice (WT: 153.53 ± 18.11; *R-Ras1* KO: 131.21 ± 18.91, *p* < 0.05; *R-Ras2* KO: 121.75 ± 14.44, *p* < 0.001 and DKO: 116.19 ± 16.42, *p* < 0.01) (**Fig. 4A**). These ratio alterations confirm that mitochondrial respiration is increased in R-Ras mutant mice, suggesting mitochondrial ATP production is increased in single and double mutant mice.

We next used the web application MetaboAnalyst®, commonly used to integrate metabolomic analysis (PMID: 29955821), to ask how the changes in metabolites we had observed affected different steps in the Krebs cycle. In both *R-Ras1* and *R-Ras2* KO mice, we found that pyruvate was significantly elevated (*R-Ras1* KO: +47.6%, *p* < 0.01; *R-Ras2* KO: +26.6%, *p* < 0.01), whereas citrate was significantly reduced (*R-Ras1* KO: -25.6% *p* < 0.05; *R-Ras2* KO: -27.0, *p <* 0.05) relative to WT (**Fig. 4*****B***). In DKO mice, several metabolites were significantly altered: pyruvate +11.6% (*p* < 0.01); lactate -17.0% (*p* < 0.05) and succinate -25.2% (*p* < 0.05) (**Fig. 4*****B***). Taken together, these data show that several metabolites directly involved in Krebs cycle metabolism are altered in *R-Ras* mutant mice (**Fig. 4*****C***) in a way that suggest that mitochondria produce more energy relative to controls.

### Example 2.6. Absence of R-Ras1 and R-Ras2 leads to degeneration and loss of axonal function.

We next sought to determine if the increased mitochondrial activity we had observed lead to an increase in reactive oxygen species (ROS). To this end, we used a commercially available kit to detect protein oxidation levels in optic nerve homogenates from adult mutant mice compared to controls (**Fig. 5*****A***). Although we did not observe significant differences in the single mutants relative to the WT, we detected presence of a significantly elevated oxidizing environment in adult DKO mice (*R-Ras1* KO: 97.7 ± 26.5%; *R-Ras2* KO: 131.8 ± 46.6% and DKO: 162.4 ± 53.4%, *p* < 0.05) (**Fig. 5*****A***).

Given the relationship between axon integrity and morphology, we next measured the axon area of the optic nerve to see if increased protein oxidation was associated with tissue degeneration in Ras mutant mice (**Fig. 5*****C***). In line with the results above, caliber in axonal sections were not statistically different in single mutant mice, but were decreased in axons in DKO mice, suggesting axonal degeneration (WT: 0.30 ± 0.22 µm²; *R-Ras1* KO: 0.26 ± 0.19 µm²; *R-Ras2* KO: 0.32 ± 0.24 µm²; DKO 0.23 ± 0.22 µm², *p <* 0.01) (**Fig. 5*****B***).

We then asked whether the degree of phosphorylation/dephosphorylation of cytoskeletal elements would allow us to assess how axonal health was affected in Ras mutant mice. To this end, we performed western blot analysis in single and double mutant adult mice with antibodies for Tau 1, neurofilament heavy chain (NFH) and PSA-NCAM. In optic nerve homogenates, we found that the phosphorylated form of Tau 1 was significantly decreased in *R-Ras2* KO and DKO mice relative to WT (*R-Ras1* KO: 75.9 ± 38.5%; *R-Ras2* KO: 47.7 ± 22.1%, *p* < 0.01 and DKO 37.4 ± 3.8%, *p* < 0.001) (**Fig. 6*****A***). In longitudinal optic nerve sections, we identified the non-phosphorylated form of NFH by immunohistochemical staining with the specific SMI-32 antibody (Thangavel et al., 2009) (**Fig. 6*****D***). Signal quantification revealed a significant increase in axonal dephosphorylation in DKO relative to WT mice (58.7 ± 25.5%, *p* < 0.001) (**Fig. 6*****B***). PSA-NCAM is commonly upregulated in myelin diseases (Charles, 2002), and we next quantified PSA-NCAM levels by immunohistochemical staining in longitudinal optic nerve sections. Similar to NFH, PSA-NCAM was significantly increased in DKO relative to WT mice (50.0 ± 12.9%, *p* < 0.01), **Fig. *6C******, E***)*.*

Cumulatively, the decrease in caliber and changes in phosphorylation levels of the axonal cytoskeleton, together with increased PSA-NCAM expression confirmed presence of axonal degeneration in the double mutants. To evaluate whether these alterations had functional consequences, we performed optokinetic reflex measurements, a test commonly used to evaluate retinal function in retinal degeneration mouse models, in control and Ras mutant mice (**Fig. 6*****F***). In this assay, experimental animals are placed in an environment with a screen simulating rotating black and white vertical bars, and in which their characteristic head reflex movements in the same direction as the rotating bars is measured. Contrast sensitivity is used to assess degree of capacity for visual discrimination. We found that reflex responses induced by bar rotation at different spatial frequencies were significantly lower in DKO mice relative to both single mutants and control animals (**Fig. 6*****G***). Specifically, contrast sensitivity in the range of 0.011-0.355 cycles/degree was significantly weaker (*p* < 0.001, two-way ANOVA, Bonferroni post-tests, *n* = 8 animals per group) in DKO mice, indicating a strong visual impairment.
Taken together, these findings suggest that myelin deficiency caused by the loss of R-Ras1 and R-Ras2 leads to increased ROS levels, which in turns causes severe axonal degeneration.

## Claims

1. Transgenic knock-out *R-Ras2*^{-/-} mouse model of myelin pathologies, or transgenic oligodendrocytes and/or neurons derived thereof, **characterized by** the disruption or inactivation of the gene *R-Ras2.*

2. Transgenic double knock-out *R-Rasl*^{-/-} and *R-Ras2*^{-/-} mouse model of myelin pathologies, or transgenic oligodendrocytes and/or neurons derived thereof, according to claim 1, **characterized by** the disruption or inactivation of the genes *R-Ras2* and *R-Ras1.*

3. Transgenic knock-out mouse, according to any of the claims 1 or 2, **characterized in that** it is a model of a myelin pathology selected from the group comprising: amyotrophic lateral sclerosis, neuromyelitis optica, multiple sclerosis, Charcot Marie Tooth disease and leukodystrophies.

4. An *in vitro* method for screening candidate compounds for treating myelin pathologies, which comprises: a) determining in the mouse model or the oligodendrocytes or neurons of claim 1 the level of expression of *R-Ras2* after administering the candidate molecule and b) where, if after administering the candidate molecule, the level of expression of *R-Ras2* is higher as compared with the level of expression determined before administering the candidate molecule, this is indicative that the candidate molecule is effective in the treatment myelin pathologies.

5. An *in vitro* method for screening candidate compounds for treating myelin pathologies, according to claim 4, which comprises: a) determining in the mouse model or the oligodendrocytes or neurons of claim 2 the level of expression of *R-Ras2* and *R-Ras1* after administering the candidate molecule and b) where, if after administering the candidate molecule, the level of expression of *R-Ras2* and *R-Ras1* is higher as compared with the level of expression determined before administering the candidate molecule, this is indicative that the candidate molecule is effective in the treatment myelin pathologies.

6. *In vitro* method, according to any of the claims 4 or 5, which further comprises determining, after the administration of the candidate molecule, whether the oligodendrocytes and/or neurons have evolved to mature oligodendrocytes and/or neurons, wherein if immature oligodendrocytes and/or neurons have evolved to mature oligodendrocytes and/or neurons, this is indicative that the candidate molecule is effective in the treatment myelin pathologies.

7. *In vitro* method, according to claim 6, wherein the determination of the maturity degree of the oligodendrocytes/neurons is carried out by determining the expression level of the following biomarkers: PDGFRalfa, A2B5, NG2, PSA-NCAM, O4, NKX2 and/or TCF7L2, wherein an increased level of expression of these biomarkers is indicative that the oligodendrocytes and/or neurons have evolved to mature oligodendrocytes and/or neurons.

8. *In vitro* method, according to any of the claims 4 or 5, which further comprises determining, after the administration of the candidate molecule, whether the oligodendrocytes and/or neurons are able to generate myelin, wherein if the oligodendrocytes and/or neurons are able to generate myelin, this is indicative that the candidate molecule is effective in the treatment myelin pathologies.

9. *In vitro* method, according to claim 8, wherein the determination whether the oligodendrocytes and/or neurons are able to generate myelin is carried out by determining the expression level of the following biomarkers: MBP, MAG, MOG, PLP and/or CNPasa, wherein an increased level of expression of these biomarkers is indicative that the oligodendrocytes and/or neurons are able to generate myelin.

10. *In vitro* method, according to any of the claims 4 to 9, wherein the myelin pathology is selected from the group comprising: amyotrophic lateral sclerosis, neuromyelitis optica, multiple sclerosis, Charcot Marie Tooth disease and leukodystrophies.

11. *In vitro* method for obtaining a transgenic mouse model of myelin pathologies, or transgenic oligodendrocytes and/or neurons derived thereof, which comprises the disruption or inactivation of the gene *R-Ras2.*

12. *In vitro* method, according to claim 11, further comprising the disruption or inactivation of the gene *R-Ras1.*

13. *In vitro* method, according to any of the claims 11 or 12, wherein the myelin pathology is selected from the group comprising: amyotrophic lateral sclerosis, neuromyelitis optica, multiple sclerosis, Charcot Marie Tooth disease and leukodystrophies.
